# EUROPEAN PATENT APPLICATION

(11) **EP 4 473 895 A1**
(43) Date of publication of application: **11.12.2024**
(21) Application number: 22924918.0
(22) Date of filing: 30.09.2022
(51) Int. Cl.: A61B 1/045

(54) **MEDICAL ASSISTANCE DEVICE, ENDOSCOPE DEVICE, MEDICAL ASSISTANCE METHOD, AND PROGRAM**

(30) Priority: 01.02.2022 JP 2022014192
(71) Applicant: FUJIFILM Corporation, Tokyo 106-8620 (JP)
(72) Inventor: TAKENOUCHI, Seiya, Ashigarakami-gun, Kanagawa 258-8538 (JP)
(74) Representative: HGF
(86) International application number: PCT/JP2022/036862
(87) International publication number: WO 2023/149013

(57) **Abstract**

A medical assistance device includes a processor. The processor is configured to acquire a plurality of endoscopic images acquired by capturing images in time series by an endoscope; identify, based on a first endoscopic image of the plurality of endoscopic images, a type of a first treatment tool included in the first endoscopic image; identify a type of a second treatment tool included in a second endoscopic image of the plurality of endoscopic images or a first internal aspect included in the second endoscopic image; and output information indicating a treatment name that is related to a first identification result and a second identification result, the first identification result being a result of identifying the type of the first treatment tool, the second identification result being a result of identifying the type of the second treatment tool or the first internal aspect.

## Description

### Technical Field

The technique of the present disclosure relates to a medical assistance device, an endoscope apparatus, a medical assistance method, and a program.

### Background Art

WO2021/140616A discloses a treatment system including a treatment tool that applies treatment energy to living tissue from an end effector in accordance with supplied electric power to incise the living tissue, an imaging device that generates a captured image showing a state in which the treatment energy is being applied to the living tissue from the end effector, and a control device having a processor that controls an operation of the treatment tool. The processor acquires the captured image, determines, based on the captured image, whether incision of the living tissue has been completed, and stops supply of electric power to the treatment tool in response to determining that incision of the living tissue has been completed.

### SUMMARY OF THE INVENTION

One embodiment according to the technique of the present disclosure provides a medical assistance device, an endoscope apparatus, a medical assistance method, and a program that are capable of realizing output of a treatment name with higher accuracy than in a case where a treatment name is output based on only the type of a first treatment tool. Solution to Problem

A first aspect according to the technique of the present disclosure is a medical assistance device including a processor. The processor is configured to acquire a plurality of endoscopic images acquired by capturing images in time series by an endoscope; identify, based on a first endoscopic image of the plurality of endoscopic images, a type of a first treatment tool included in the first endoscopic image; identify a type of a second treatment tool included in a second endoscopic image of the plurality of endoscopic images or a first internal aspect included in the second endoscopic image; and output information indicating a treatment name that is related to a first identification result and a second identification result, the first identification result being a result of identifying the type of the first treatment tool, the second identification result being a result of identifying the type of the second treatment tool or the first internal aspect.

A second aspect according to the technique of the present disclosure is the medical assistance device according to the first aspect, in which the second endoscopic image is an image acquired after the first endoscopic image by the endoscope.

A third aspect according to the technique of the present disclosure is the medical assistance device according to the first aspect or the second aspect, in which the processor is configured to identify, based on the first identification result and the second endoscopic image, the first internal aspect.

A fourth aspect according to the technique of the present disclosure is the medical assistance device according to any one of the first aspect to the third aspect, in which the processor is configured to, in a case where the type of the first treatment tool is not identified in a plurality of frames after the type of the first treatment tool is identified, identify the type of the second treatment tool or the first internal aspect within a time period determined in accordance with the type of the first treatment tool.

A fifth aspect according to the technique of the present disclosure is the medical assistance device according to any one of the first aspect to the fourth aspect, in which the processor is configured to output an output image associated with the treatment name, and the output image is an image acquired between the first endoscopic image and the second endoscopic image of the plurality of images.

A sixth aspect according to the technique of the present disclosure is the medical assistance device according to any one of the first aspect to the fifth aspect, in which the processor is configured to further identify a type of a third treatment tool included in a third endoscopic image or a second internal aspect included in the third endoscopic image, and the information indicating the treatment name is information that is related to the first identification result, the second identification result, and a third identification result, the third identification result being a result of identifying the type of the third treatment tool or the second internal aspect.

A seventh aspect according to the technique of the present disclosure is the medical assistance device according to the sixth aspect, in which the second identification result is a result of identifying the type of the second treatment tool.

An eighth aspect according to the technique of the present disclosure is the medical assistance device according to the sixth aspect, in which the second identification result is a result of identifying the first internal aspect.

A ninth aspect according to the technique of the present disclosure is the medical assistance device according to any one of the sixth aspect to the eighth aspect, in which the third endoscopic image is an image acquired after the second endoscopic image by the endoscope.

A tenth aspect according to the technique of the present disclosure is the medical assistance device according to the first aspect, in which the first endoscopic image is an image acquired after the second endoscopic image by the endoscope.

An eleventh aspect according to the technique of the present disclosure is the medical assistance device according to the tenth aspect, in which the first internal aspect is a treatment target region.

A twelfth aspect according to the technique of the present disclosure is the medical assistance device according to any one of the first aspect to the eleventh aspect, in which the first internal aspect includes a lesion region, a burn scar, a resection region, a change in the resection region, a hump region, and/or a marked region.

A thirteenth aspect according to the technique of the present disclosure is the medical assistance device according to any one of the first aspect to the twelfth aspect, in which the first internal aspect includes a lesion region, and the information indicating the treatment name is information that is based on a size of the first treatment tool and a size of the lesion region.

A fourteenth aspect according to the technique of the present disclosure is an endoscope apparatus including the medical assistance device according to any one of the first aspect to the thirteenth aspect and the endoscope.

A fifteenth aspect according to the technique of the present disclosure is a medical assistance method including acquiring a plurality of endoscopic images acquired by capturing images in time series by an endoscope; identifying, based on a first endoscopic image of the plurality of endoscopic images, a type of a first treatment tool included in the first endoscopic image; identifying a type of a second treatment tool included in a second endoscopic image of the plurality of endoscopic images or a first internal aspect included in the second endoscopic image; and outputting information indicating a treatment name that is related to a first identification result and a second identification result, the first identification result being a result of identifying the type of the first treatment tool, the second identification result being a result of identifying the type of the second treatment tool or the first internal aspect.

A sixteenth aspect according to the technique of the present disclosure is a program for causing a computer to execute a process including acquiring a plurality of endoscopic images acquired by capturing images in time series by an endoscope; identifying, based on a first endoscopic image of the plurality of endoscopic images, a type of a first treatment tool included in the first endoscopic image; identifying a type of a second treatment tool included in a second endoscopic image of the plurality of endoscopic images or a first internal aspect included in the second endoscopic image; and outputting information indicating a treatment name that is related to a first identification result and a second identification result, the first identification result being a result of identifying the type of the first treatment tool, the second identification result being a result of identifying the type of the second treatment tool or the first internal aspect.

### BRIEF DESCRIPTION OF THE DRAWINGS

Fig. 1 is a conceptual diagram illustrating an example of the overall configuration of an endoscope apparatus;
Fig. 2 is a block diagram illustrating an example of the hardware configuration of the endoscope apparatus;
Fig. 3 is a block diagram illustrating an example of the functions of a main part of a processor;
Fig. 4 is a conceptual diagram illustrating an example of the details of a process performed by a treatment tool identification unit;
Fig. 5 is a conceptual diagram illustrating an example of the details of a process performed by the treatment tool identification unit and an internal aspect identification unit;
Fig. 6 is a conceptual diagram illustrating an example of the details of a process performed by the internal aspect identification unit;
Fig. 7 is a conceptual diagram illustrating an example of the details of a process performed by a treatment name identification unit;
Fig. 8 is a conceptual diagram illustrating an example of the details of a process performed by an output unit;
Fig. 9 is a flowchart illustrating an example of the flow of a treatment name identification process;
Fig. 10 is a conceptual diagram illustrating an example of the details of a process performed by the treatment name identification unit;
Fig. 11 is a conceptual diagram illustrating an example of the details of a process performed by the treatment name identification unit;
Fig. 12 is a conceptual diagram illustrating an example of the details of a process performed by the treatment name identification unit;
Fig. 13 is a conceptual diagram illustrating an example of the details of a process performed by the internal aspect identification unit;
Fig. 14 is a conceptual diagram illustrating an example of the details of a process performed by the treatment tool identification unit;
Fig. 15 is a conceptual diagram illustrating an example of the details of a process performed by the treatment name identification unit;
Fig. 16 is a conceptual diagram illustrating an example of the details of a process performed by the treatment tool identification unit and the internal aspect identification unit;
Fig. 17 is a conceptual diagram illustrating an example of the details of a process performed by the treatment name identification unit;
Fig. 18 is a conceptual diagram illustrating an example of the details of a process performed by the treatment tool identification unit and the internal aspect identification unit;
Fig. 19 is a conceptual diagram illustrating an example of the details of a process performed by the treatment name identification unit;
Fig. 20 is a conceptual diagram illustrating an example of the details of a process performed by the treatment tool identification unit and the internal aspect identification unit;
Fig. 21 is a conceptual diagram illustrating an example of the details of a process performed by the treatment name identification unit; and
Fig. 22 is a conceptual diagram illustrating an example of the overall configuration of an endoscope system.

### DESCRIPTION OF EMBODIMENTS

Hereinafter, an example of embodiments of a medical assistance device, an endoscope apparatus, a medical assistance method, and a program according to the technique of the present disclosure will be described with reference to the accompanying drawings.

First, terms used in the following description will be described.

CPU is an abbreviation of "Central Processing Unit". GPU is an abbreviation of "Graphics Processing Unit". RAM is an abbreviation of "Random Access Memory". EEPROM is an abbreviation of "Electrically Erasable Programmable Read-Only Memory". ASIC is an abbreviation of "Application Specific Integrated Circuit". PLD is an abbreviation of "Programmable Logic Device". FPGA is an abbreviation of "Field-Programmable Gate Array". SoC is an abbreviation of "System-on-a-chip". SSD is an abbreviation of "Solid State Drive". USB is an abbreviation of "Universal Serial Bus". HDD is an abbreviation of "Hard Disk Drive". EL is an abbreviation of "Electro-Luminescence". I/F is an abbreviation of "Interface". CMOS is an abbreviation of "Complementary Metal Oxide Semiconductor". CCD is an abbreviation of "Charge Coupled Device". NN is an abbreviation of "Neural Network". EMR is an abbreviation of "Endoscopic Mucosal Resection". ESD is an abbreviation of "Endoscopic Submucosal Dissection". AI is an abbreviation of "Artificial Intelligence". PIP is an abbreviation of "Picture-In-Picture".

### First Embodiment

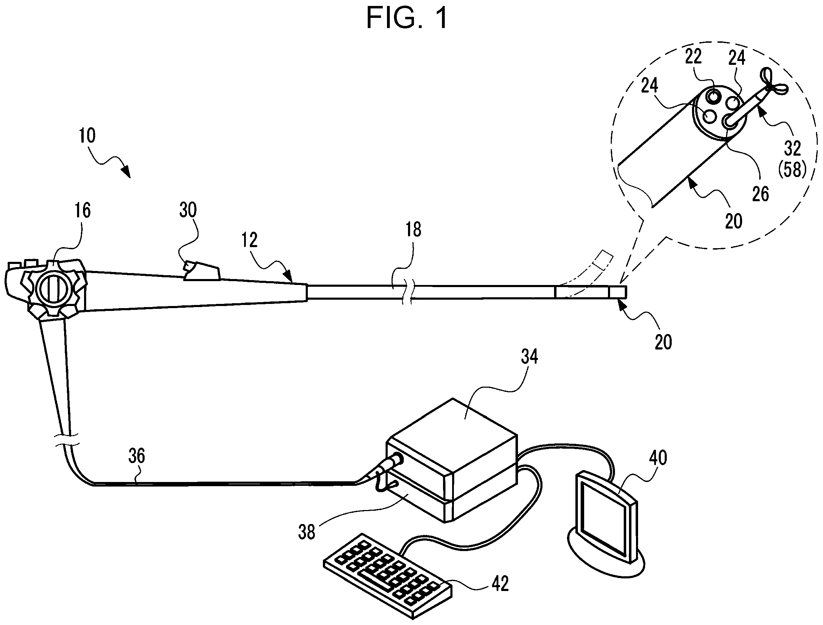

As illustrated as an example in Fig. 1, an endoscope apparatus 10 includes an endoscope unit 12. The endoscope apparatus 10 is an apparatus for performing diagnosis and treatment on the inside of a body (for example, the stomach, large intestine, or the like) via the endoscope unit 12. The endoscope unit 12 includes an operation section 16 and an insertion section 18. The insertion section 18 is partially bent as a result of the operation section 16 being operated. As a result, the insertion section 18 is inserted while being bent in accordance with the shape of the inside of the body (for example, the shape of the large intestine tube). The endoscope apparatus 10 is an example of an "endoscope apparatus" according to the technique of the present disclosure.

A distal end portion 20 of the insertion section 18 is provided with an endoscope 22, an illumination device 24, and a treatment tool opening 26. The endoscope 22 is a device that captures an image of the inside of a body. An example of the endoscope 22 is a CMOS camera. However, this is merely an example, and another type of camera, such as a CCD camera, may be used. The illumination device 24 is a device that irradiates the inside of a body with illumination light. The treatment tool opening 26 is an opening for causing a treatment tool 58 to protrude from the distal end portion 20. The treatment tool 58 is inserted into the insertion section 18 through a treatment tool insertion port 30. The treatment tool 58 passes through the insertion section 18 and is protruded into the body from the treatment tool opening 26. In the example illustrated in Fig. 1, forceps 32 protrude, as the treatment tool 58, from the treatment tool opening 26. The endoscope 22 is an example of an "endoscope" according to the technique of the present disclosure.

The endoscope apparatus 10 includes a control device 34. The endoscope unit 12 is electrically connected to the control device 34 via a cable 36. The endoscope unit 12 is optically connected to a light source device 38 via the cable 36. A display device 40 and a reception device 42 are connected to the control device 34.

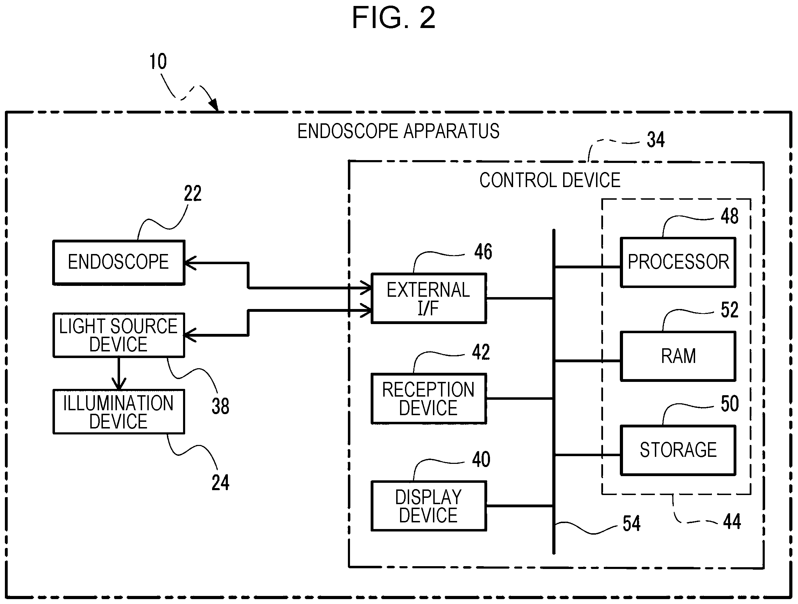

As illustrated as an example in Fig. 2, the control device 34 includes a computer 44 and an external I/F 46. The computer 44 has a processor 48, a storage 50, and a RAM 52. The processor 48, the storage 50, the RAM 52, the external I/F 46, the display device 40, and the reception device 42 are connected to a bus 54. The control device 34 is an example of a "medical assistance device" according to the technique of the present disclosure. The computer 44 is an example of a "computer" according to the technique of the present disclosure. The processor 48 is an example of a "processor" according to the technique of the present disclosure.

The processor 48 has, for example, a CPU and a GPU, and controls the entire control device 34. The GPU operates under control by the CPU and executes screen display, image processing, and/or the like. The processor 48 may be one or more CPUs having a GPU function integrated thereto, or may be one or more CPUs not having a GPU function integrated thereto.

The storage 50 is a nonvolatile storage device that stores various programs, various parameters, and the like. An example of the storage 50 is a flash memory (for example, an EEPROM and/or an SSD). The flash memory is merely an example. Another nonvolatile storage device such as an HDD, or a combination of two or more types of nonvolatile storage devices may be used. The RAM 52 is a memory that temporarily stores information and is used as a work memory by the processor 48.

The external I/F 46 manages communication between the control device 34 and the endoscope 22. For example, the external I/F 46 outputs a signal from the processor 48 to the endoscope 22, and outputs a signal from the endoscope 22 to the processor 48. The processor 48 acquires, via the external I/F 46, images acquired by capturing images of the inside of a body by the endoscope 22 (hereinafter simply referred to as "endoscopic images"). The endoscopic images according to the present embodiment may be moving images (for example, live view images, moving images for recording, or the like). However, this is merely an example, and endoscopic images 56 may be, for example, still images or the like acquired through continuous shooting. The external I/F 46 manages communication between the control device 34 and the light source device 38. The processor 48 controls the light source device 38 via the external I/F 46. The light source device 38 supplies illumination light to the illumination device 24 in accordance with an instruction from the processor 48.

The display device 40 displays various pieces of information (for example, images, characters, and the like) under control by the processor 48. The display device 40 is, for example, an EL display, a liquid crystal display, or the like.

The reception device 42 receives an instruction from a user of the endoscope apparatus 10. The user of the endoscope apparatus 10 is, for example, a medical practitioner (for example, a doctor, a nurse, a technician, and/or the like). The instruction received by the reception device 42 is acquired by the processor 48. The reception device 42 is, for example, a keyboard and/or a mouse. The keyboard and/or the mouse is merely an example. At least one of a proximity input device that receives a proximity input, a sound input device that receives a sound input, or a gesture input device that receives a gesture input may be applied as the reception device 42 instead of the keyboard and/or the mouse or together with the keyboard and/or the mouse. The proximity input device is, for example, a touch panel, a tablet, or the like. The reception device 42 and the endoscope apparatus 10 may be connected to each other in a wired manner or a wireless manner.

In the case of performing a treatment (for example, examination, remedy, or the like) using the endoscope apparatus 10, it is a great operation burden for a medical practitioner to record a treatment name on a medical record or the like every time he/she performs a treatment on a lesion. In particular, when treatments are performed on a plurality of lesions, it is predicted that the operation burden on the medical practitioner will be further increased. On the other hand, an image recognition process may be performed on an endoscopic image during a treatment to specify a treatment name. However, there are various types of treatments, and there is room for improvement in accurately identifying a treatment name.

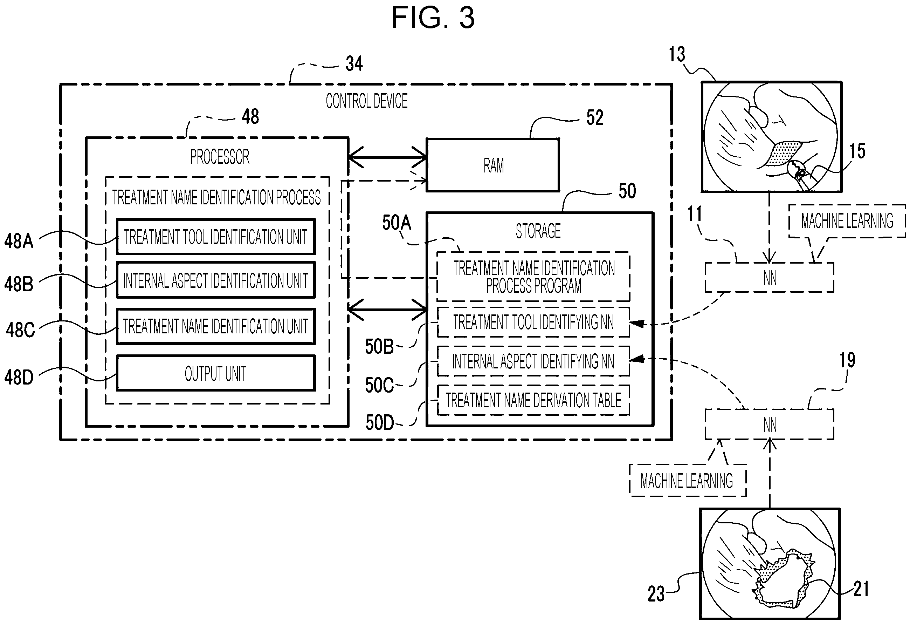

In view of these circumstances, in the present embodiment, the processor 48 performs a treatment name identification process as illustrated as an example in Fig. 3. The storage 50 stores a treatment name identification process program 50A. The processor 48 reads out the treatment name identification process program 50A from the storage 50 and executes the read treatment name identification process program 50A on the RAM 52, thereby performing a treatment name identification process. The treatment name identification process is implemented by the processor 48 operating as a treatment tool identification unit 48A, an internal aspect identification unit 48B, a treatment name identification unit 48C, and an output unit 48D. The treatment name identification process program 50A is an example of a "program" according to the technique of the present disclosure.

The storage 50 also stores a treatment tool identifying NN 50B, an internal aspect identifying NN 50C, and a treatment name derivation table 50D. The treatment tool identifying NN 50B and the internal aspect identifying NN 50C are trained models generated by optimizing an NN 11 and an NN 19 through machine learning. The treatment tool identifying NN 50B is acquired by causing the NN 11 to perform machine learning. The machine learning that the NN 11 is caused to perform is machine learning using a plurality of treatment tool images 13. An example of the treatment tool image 13 is an endoscopic image including a treatment tool 15. For example, the treatment tool 15 refers to a treatment tool (for example, forceps, a snare, a knife, and/or the like) that is used while being protruded from a distal end portion (for example, the distal end portion 20) of an endoscope. In the example illustrated in Fig. 3, forceps are illustrated as the treatment tool 15. In the present embodiment, the processor 48 uses the treatment tool identifying NN 50B to identify a treatment tool included in an endoscopic image, that is, a treatment tool corresponding to the treatment tool 15. In the present embodiment, a treatment tool included in an endoscopic image refers to a treatment tool included as an image in the endoscopic image.

The internal aspect identifying NN 50C is a trained model generated by optimizing the NN 19 through machine learning. The internal aspect identifying NN 50C is acquired by causing the NN 19 to perform machine learning. The machine learning that the NN 19 is caused to perform is machine learning using a plurality of internal aspect images 23. An example of the internal aspect image 23 is an endoscopic image including an internal aspect 21. The internal aspect 21 refers to an aspect indicating a scar (for example, a resection scar and/a burn scar) resulting from a treatment performed in the body using a treatment tool protruded from a distal end portion (for example, the distal end portion 20) of an endoscope. In the example illustrated in Fig. 3, a burn scar is illustrated as the internal aspect 21. In the present embodiment, the processor 48 uses the internal aspect identifying NN 50C to identify an internal aspect included in an endoscopic image, that is, an internal aspect corresponding to the internal aspect 21.

The treatment name derivation table 50D is a table for deriving a treatment name. The treatment name derivation table 50D is a table having, as input values, the type of a treatment tool included in an endoscopic image and an internal aspect included in the endoscopic image, and having, as an output value, a treatment name. Instead of the table, an arithmetic expression may be used in which a value indicating the type of a treatment tool and an internal aspect is an independent variable and a value indicating a treatment name is a dependent variable.

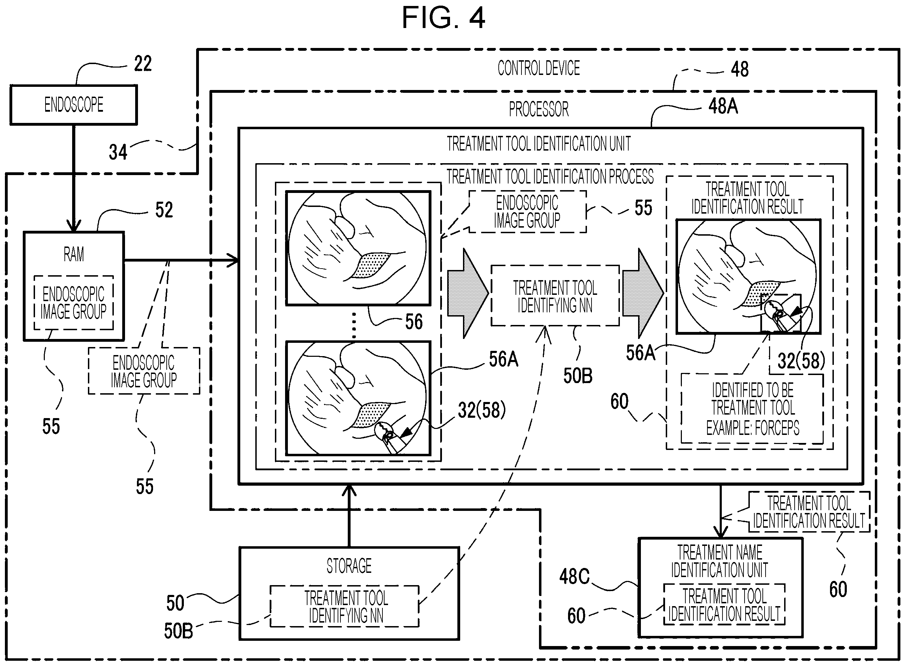

As illustrated as an example in Fig. 4, first, images of the inside of a body is captured in time series by the endoscope 22, and thereby an endoscopic image group 55 is acquired. The endoscopic image group 55 includes a plurality of endoscopic images 56. In the control device 34, the endoscopic image group 55 is temporarily stored in the RAM 52. The treatment tool identification unit 48A acquires the endoscopic image group 55 from the RAM 52. In this case, the treatment tool identification unit 48A may acquire several frames (for example, three frames) of endoscopic images 56 included in the endoscopic image group 55 at one time, or may consecutively acquire the endoscopic images 56 frame by frame.

The endoscopic image group 55 includes an endoscopic image 56A in which the treatment tool 58 is seen. The treatment tool identification unit 48A identifies, based on the endoscopic image 56A, the type of the treatment tool 58 included in the endoscopic image 56A. For example, the treatment tool identification unit 48A performs an image recognition process using AI to identify the type of the treatment tool 58. The endoscopic image group 55 is an example of "a plurality of endoscopic images" according to the technique of the present disclosure, and the endoscopic image 56A is an example of a "first endoscopic image" according to the technique of the present disclosure.

In a case where the treatment tool identification unit 48A performs an image recognition process using AI, the treatment tool identification unit 48A first acquires the treatment tool identifying NN 50B from the storage 50. The treatment tool identification unit 48A inputs the endoscopic images 56 to the treatment tool identifying NN 50B. Upon the endoscopic images 56 being input, the treatment tool identifying NN 50B performs identification of the type of the treatment tool 58 on the endoscopic images 56. The treatment tool identifying NN 50B outputs the type of the treatment tool 58 as a treatment tool identification result 60. Fig. 4 illustrates an example in which the type of the treatment tool 58 is identified to be forceps 32 as the treatment tool identification result 60. The treatment name identification unit 48C acquires the treatment tool identification result 60, which is a result of identifying the type of the treatment tool 58, from the treatment tool identification unit 48A. The treatment tool 58 is an example of a "first treatment tool" according to the technique of the present disclosure, and the treatment tool identification result 60 is an example of a "first identification result" according to the technique of the present disclosure.

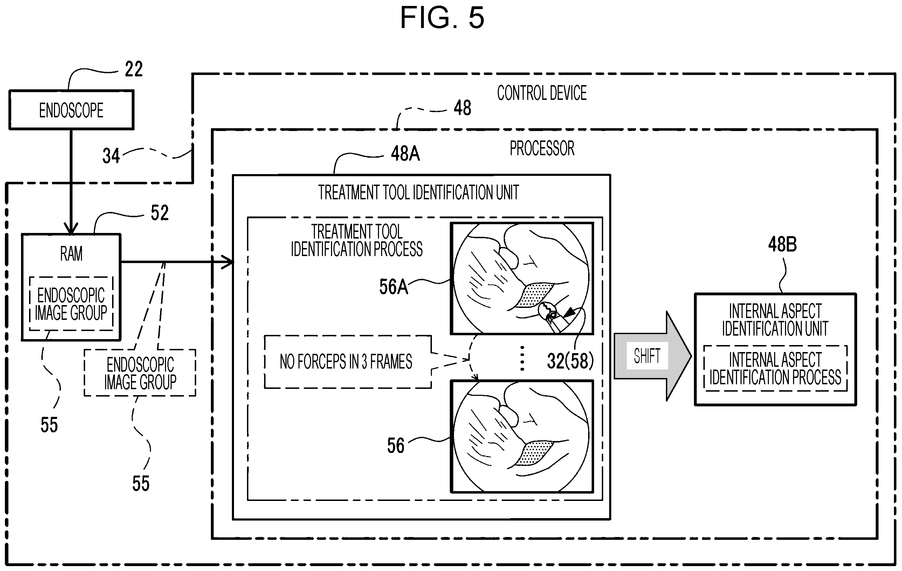

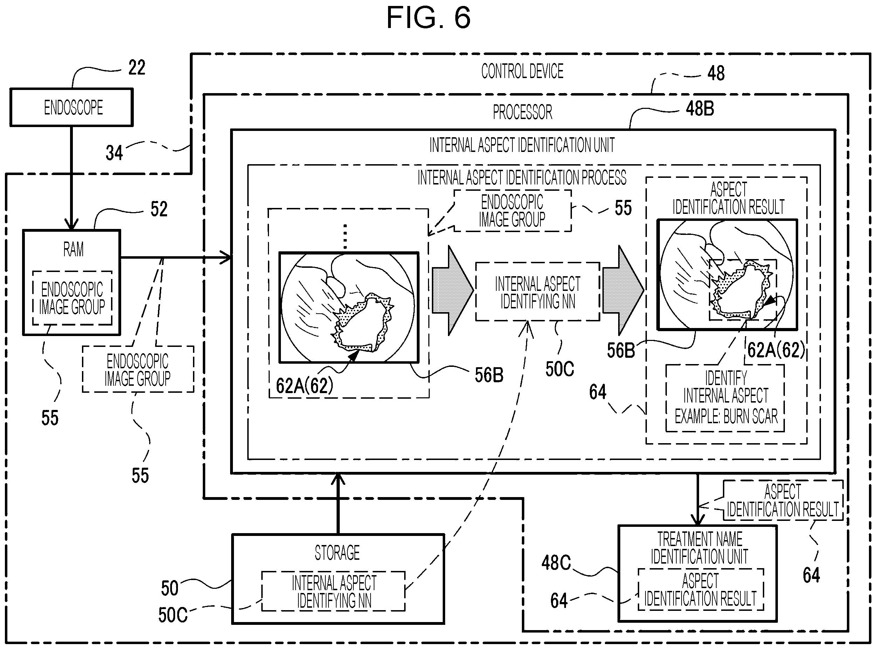

As illustrated as an example in Fig. 5, in a case where a predetermined condition is satisfied after a treatment tool identification process is performed by the treatment tool identification unit 48A, a shift from the treatment tool identification process by the treatment tool identification unit 48A to an identification process for an internal aspect 62 (see Fig. 6) by the internal aspect identification unit 48B occurs. For example, the predetermined condition is a case where the type of the treatment tool 58 is not identified in a plurality of frames (for example, two to three frames) after the type of the treatment tool 58 is identified (i.e., a case where the treatment tool 58 is not seen in the endoscopic images 56). Fig. 5 illustrates an example in which a shift to an internal aspect identification process occurs when three frames have passed from when the forceps 32 serving as the treatment tool 58 are no longer seen in the endoscopic images 56, as the predetermined condition. The internal aspect identification process refers to a process of identifying an internal aspect.

The identification of the internal aspect 62 by the internal aspect identification unit 48B is performed within a time period determined in accordance with the type of the treatment tool 58. For example, the internal aspect identification unit 48B derives a time period for identifying the internal aspect 62 by using an aspect identification time computing expression (not illustrated). The aspect identification time computing expression is a computing expression having, as an independent variable, a numerical value indicating the treatment tool identification result 60, and having, as a dependent variable, a numerical value indicating a time period for identifying the internal aspect 62. For example, in a case where the forceps 32 are identified as the treatment tool 58, the identification of the internal aspect 62 is performed within 30 seconds from the start of the identification of the internal aspect 62.

As illustrated as an example in Fig. 6, the internal aspect identification unit 48B acquires the endoscopic image group 55 from the RAM 52. The endoscopic image group 55 includes an endoscopic image 56B in which the internal aspect 62 (for example, a scar resulting from a treatment with the treatment tool 58) is seen. Here, the internal aspect 62 is generated after the treatment with the treatment tool 58. Thus, the endoscopic image 56B is an image acquired after the endoscopic image 56A.

The internal aspect identification unit 48B identifies, based on the endoscopic image 56B, the internal aspect 62 included in the endoscopic image 56B. For example, the internal aspect identification unit 48B performs an image recognition process using AI to identify the internal aspect 62. The endoscopic image 56B is an example of a "second endoscopic image" according to the technique of the present disclosure, the internal aspect 62 is an example of an "internal aspect" according to the technique of the present disclosure, and an aspect identification result 64 is an example of a "second identification result" according to the technique of the present disclosure.

In a case where the internal aspect identification unit 48B performs an image recognition process using AI, the internal aspect identification unit 48B acquires the internal aspect identifying NN 50C from the storage 50. The internal aspect identification unit 48B inputs the endoscopic images 56 to the internal aspect identifying NN 50C. Upon the endoscopic images 56 being input, the internal aspect identifying NN 50C performs identification of the internal aspect 62 on the endoscopic images 56. The internal aspect identifying NN 50C outputs the aspect identification result 64. Fig. 6 illustrates an example in which the internal aspect 62 is identified to be a burn scar 62A (i.e., a scar of a part of the inside of the body burned off by the treatment tool 58) as the aspect identification result 64. The burn scar 62A as the internal aspect 62 is merely an example, and may be, for example, smoke or the like generated when a part of the inside of the body is burned off. The treatment name identification unit 48C acquires the aspect identification result 64, which is a result of identifying the internal aspect, from the internal aspect identification unit 48B.

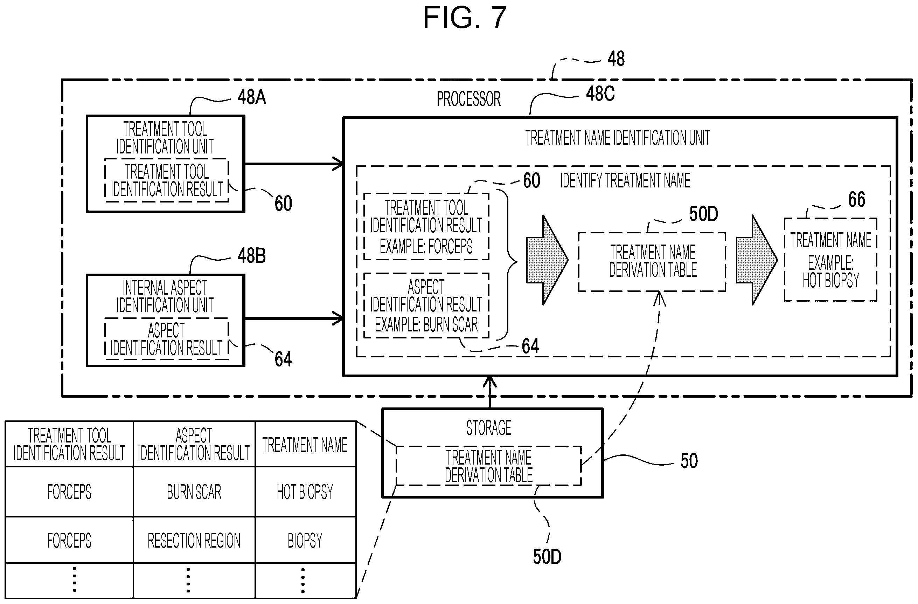

As illustrated as an example in Fig. 7, the treatment name identification unit 48C acquires the treatment tool identification result 60 from the treatment tool identification unit 48A. The treatment name identification unit 48C also acquires the aspect identification result 64 from the internal aspect identification unit 48B. The treatment name identification unit 48C identifies a treatment name 66 that is related to the treatment tool identification result 60 and the aspect identification result 64. The treatment name identification unit 48C acquires the treatment name derivation table 50D from the storage 50. The treatment name derivation table 50D is a table having the treatment tool identification result 60 and the aspect identification result 64 as input values and having the treatment name 66 as an output value. The treatment name identification unit 48C derives the treatment name 66 that is related to the treatment tool identification result 60 and the aspect identification result 64 from the treatment name derivation table 50D. In the example illustrated in Fig. 7, "forceps" are identified as the treatment tool identification result 60, and "burn scar" is identified as the aspect identification result 64. The treatment name identification unit 48C derives, from the treatment name derivation table 50D, "hot biopsy" as the treatment name 66 that is related to "forceps", which is the treatment tool identification result 60, and "burn scar", which is the aspect identification result 64. The treatment name 66 is an example of a "treatment name" according to the technique of the present disclosure.

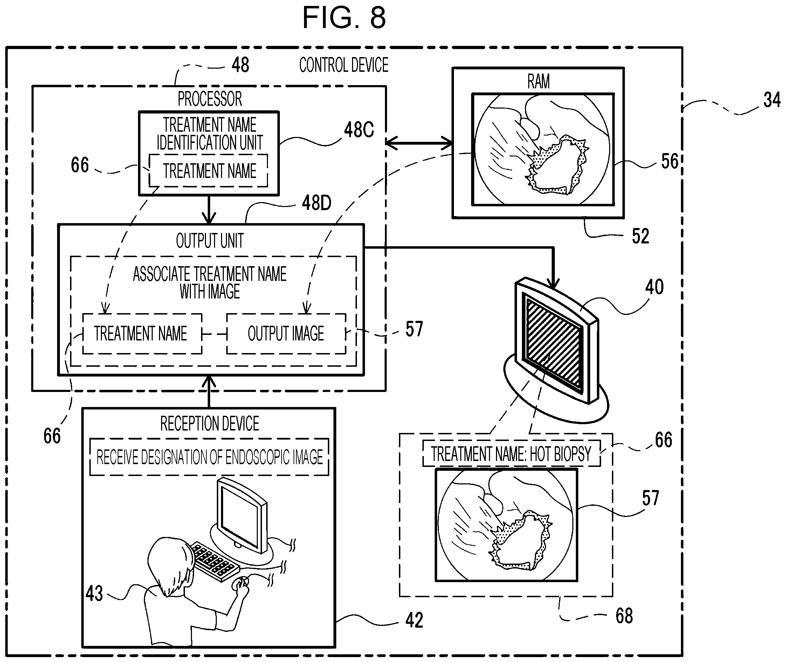

As illustrated as an example in Fig. 8, the output unit 48D acquires information indicating the treatment name 66 from the treatment name identification unit 48C. The output unit 48D also receives designation of an endoscopic image 56 by a user 43 via the reception device 42. The output unit 48D acquires the endoscopic image 56 designated by the user 43 from the RAM 52. The output unit 48D regards the acquired endoscopic image 56 as an output image 57. For example, the output image 57 is the endoscopic image 56 that is included in the endoscopic image group 55 and that is acquired during a period in which the endoscopic image 56A including the treatment tool 58 and the endoscopic image 56B including the internal aspect 62 are acquired. The output image 57 is an example of an "output image" according to the technique of the present disclosure.

The output unit 48D associates the treatment name 66 acquired from the treatment name identification unit 48C with the output image 57. For example, the output unit 48D adds information indicating the treatment name 66 as accessory information of the output image 57. The output unit 48D outputs the information indicating the treatment name 66 to the display device 40. For example, the information indicating the treatment name 66 is output as a screen 68 to the display device 40. In this case, the output unit 48D causes the display device 40 to display the screen 68. On the screen 68, the treatment name 66 and the output image 57 are displayed together. In the example illustrated in Fig. 8, a state is illustrated in which the treatment name 66 and the output image 57 are arranged and displayed within the screen 68. The treatment name 66 may be displayed in a PIP manner or a pop-up manner with respect to the output image 57.

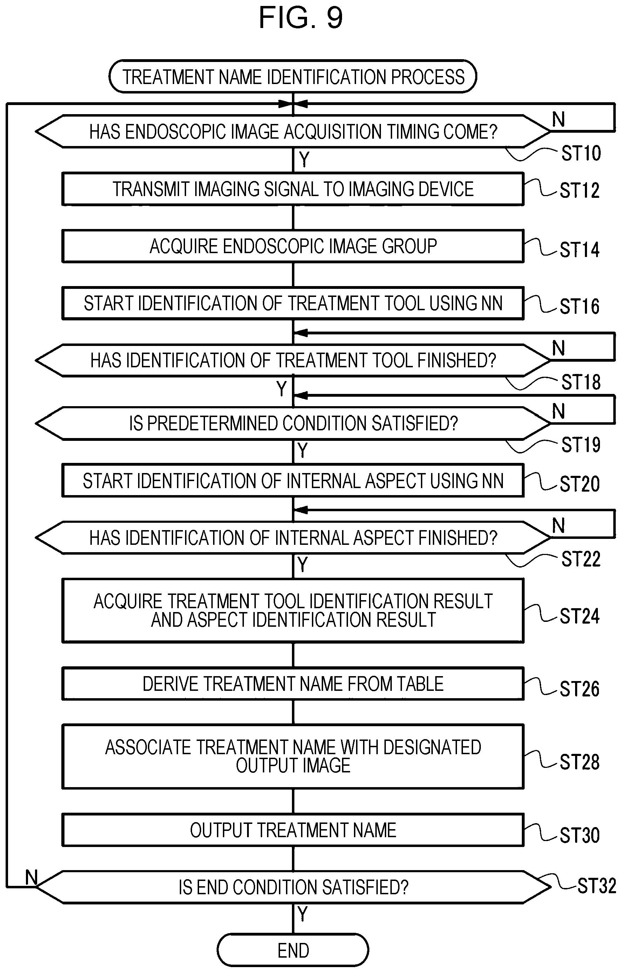

Next, the operation of the endoscope apparatus 10 will be described with reference to Fig. 9. Fig. 9 illustrates an example of a flow of a treatment name identification process performed by the processor 48 of the endoscope apparatus 10. The treatment name identification process illustrated in Fig. 9 is an example of a "medical assistance method" according to the technique of the present disclosure.

In the treatment name identification process illustrated in Fig. 9, first, in step ST10, the treatment tool identification unit 48A determines whether a timing to acquire the endoscopic images 56 has come. If the timing to acquire the endoscopic images 56 has not come in step ST10, a negative determination is made and a determination in step ST10 is made again. If the timing to acquire the endoscopic images 56 has come in step ST10, an affirmative determination is made and the treatment name identification process proceeds to step ST12.

In step ST12, the treatment tool identification unit 48A transmits an imaging signal to the endoscope 22. After the process of step ST12 is executed, the treatment name identification process proceeds to step ST14.

In step ST 14, the treatment tool identification unit 48A acquires the endoscopic image group 55 captured by the endoscope 22 from the RAM 52. After the process of step ST14 is executed, the treatment name identification process proceeds to step ST16.

In step ST16, the treatment tool identification unit 48A starts identification of the type of the treatment tool 58 included in the endoscopic image 56A by using the treatment tool identifying NN 50B for the endoscopic images 56 acquired in step ST14. After the process of step ST16 is executed, the treatment name identification process proceeds to step ST18.

In step ST 18, the treatment tool identification unit 48A determines whether identification of the type of the treatment tool 58 has finished. If identification of the type of the treatment tool 58 has not finished in step ST18, a negative determination is made and a determination in step ST18 is made again. If identification of the type of the treatment tool 58 has finished in step ST18, an affirmative determination is made and the treatment name identification process proceeds to step ST19.

In step ST19, the internal aspect identification unit 48B determines whether a predetermined condition is satisfied. An example of a timing to identify an internal aspect is a timing at which three frames have passed after the treatment tool 58 is not seen in the endoscopic images 56 any longer as a result of the process of step ST16 and the process of step ST18. If the predetermined condition is not satisfied in step ST19, a negative determination is made and a determination in step ST19 is made again. If the predetermined condition is satisfied in step ST19, an affirmative determination is made and the process name identification process proceeds to step ST20.

In step ST20, the internal aspect identification unit 48B starts identification of the internal aspect 62 included in the endoscopic image 56B by using the internal aspect identifying NN 50C for the endoscopic images 56 acquired in step ST14. After the process of step ST20 is executed, the treatment name identification process proceeds to step ST22.

In step ST22, the internal aspect identification unit 48B determines whether identification of the internal aspect 62 has finished. If identification of the internal aspect 62 has not finished in step ST22, a negative determination is made and a determination in step ST22 is made again. If identification of the internal aspect 62 has finished in step ST22, an affirmative determination is made and the treatment name identification process proceeds to step ST24.

In step ST24, the treatment name identification unit 48C acquires the treatment tool identification result 60 from the treatment tool identification unit 48A. The treatment name identification unit 48C also acquires the aspect identification result 64 from the internal aspect identification unit 48B. After the process of step ST24 is executed, the treatment name identification treatment proceeds to step ST26.

In step ST26, the treatment name identification unit 48C derives, from the treatment name derivation table 50D, the treatment name 66 that is related to the treatment tool identification result 60 and the aspect identification result 64. After the process of step ST26 is executed, the treatment name identification process proceeds to step ST28.

In step ST28, the output unit 48D associates the treatment name 66 acquired from the treatment name identification unit 48C with the output image 57 designated by the user 43 via the reception device 42. After the process of step ST28 is executed, the treatment name identification process proceeds to step ST30.

In step ST30, the output unit 48D outputs, as information indicating the treatment name 66, the screen 68 including the treatment name 66 and the output image 57 to the display device 40. Accordingly, the treatment name 66 and the output image 57 are arranged and displayed on the display device 40. After the process of step ST30 is executed, the treatment name identification process proceeds to step ST32.

In step ST32, the output unit 48D determines whether a condition for ending the treatment name identification process (hereinafter referred to as an "end condition") is satisfied. An example of the end condition is a condition that an instruction to end the treatment name identification process has been received by the reception device 42. If the end condition is not satisfied in step ST32, a negative determination is made and the treatment name identification process returns to step ST10. If the end condition is satisfied in step ST32, an affirmative determination is made and the treatment name identification process ends.

As described above, in the endoscope apparatus 10 according to the first embodiment, information indicating the treatment name 66 is output based on the treatment tool identification result 60, which is a result of identifying the type of the treatment tool 58, and the aspect identification result 64, which is a result of identifying the internal aspect 62. Thus, according to this configuration, it is possible to realize output of the treatment name 66 with higher accuracy than in a case where the treatment name 66 is output based on only a result of identifying the type of the treatment tool 58.

In the endoscope apparatus 10 according to the first embodiment, the endoscopic image 56B including the internal aspect 62 is acquired by the endoscope 22 after the endoscopic image 56A including the treatment tool 58 is acquired. Thus, according to this configuration, it is possible to realize output of the treatment name 66 with higher accuracy than in a case where the order in which the endoscopic image 56A and the endoscopic image 56B are acquired is not taken into consideration in the case of identifying the treatment name 66.

In the endoscope apparatus 10 according to the first embodiment, in a case where the type of the treatment tool 58 is not identified in a plurality of frames (for example, two to three frames) after the type of the treatment tool 58 is identified, a shift from the treatment tool identification process by the treatment tool identification unit 48A to the internal aspect identification process by the internal aspect identification unit 48B occurs. The identification of the internal aspect 62 by the internal aspect identification unit 48B is performed within a time period determined in accordance with the type of the treatment tool 58. Thus, according to this configuration, it is possible to realize output of the treatment name 66 with higher accuracy than in a case where the time period from when the type of the treatment tool 58 is identified to when the internal aspect 62 is identified is not taken into consideration.

For example, after the treatment tool 58 is identified, within a predetermined time period from when the treatment tool 58 is not seen in the endoscopic image 56A any longer, the subsequent identification of the internal aspect 62 is performed. This makes it easy to estimate that the treatment with the treatment tool 58 and the internal aspect 62 as a result thereof are included in a series of treatment actions, and the treatment name 66 can be accurately identified.

In the endoscope apparatus 10 according to the first embodiment, the treatment name 66 is associated with the output image 57, and the output image 57 is an image acquired while the endoscopic image 56A and the endoscopic image 56B are acquired. Thus, according to this configuration, it is possible to realize an improvement in the relevance between the treatment name 66 and the endoscopic image 56 as compared with a case where the treatment name 66 is associated with the endoscopic image 56 that does not include the treatment tool 58 or the internal aspect 62.

In the above-described embodiment, a description has been given of an example in which the treatment name 66 and the output image 57 are associated with each other, but the technique of the present disclosure is not limited thereto. For example, the treatment name 66 may be associated with the endoscopic image 56B including the internal aspect 62, or may be associated with a plurality of frames of endoscopic images including the endoscopic image 56B.

In the above-described embodiment, an example has been given in which information indicating the screen 68 having the treatment name 66 and the output image 57 displayed together thereon is output to the display device 40, but this is merely an example. Instead of or together with the visible display of the screen 68 in the display device 40, audible indication using a speaker or permanent visible display using a paper medium or the like may be performed. In addition, for example, information indicating the treatment name 66 and output from the output unit 48D may be stored in the storage 50.

In the above-described embodiment, a description has been given of an example in which the type of the treatment tool 58 is identified by using the treatment tool identifying NN 50B and the internal aspect 62 is identified by using the internal aspect identifying NN 50C, but the technique of the present disclosure is not limited thereto. For example, an image identifying NN (not illustrated) may be used instead of the treatment tool identifying NN 50B and the internal aspect identifying NN 50C. The image identifying NN is an NN for identifying the treatment tool 58 and the internal aspect 62 included in the endoscopic image 56A, and is a trained model generated by optimizing an NN through machine learning. The treatment tool identification unit 48A identifies the type of the treatment tool 58 included in the endoscopic image 56A by using the image identifying NN. The internal aspect identification unit 48B identifies the internal aspect 62 included in the endoscopic image 56B by using the image identifying NN.

### Modification 1

In the above-described embodiment, a description has been given of an example in which the treatment name derivation table 50D is a table having, as the treatment tool identification result 60, forceps as an input value, having, as the aspect identification result 64, burn scar or resection region as an input value, and having, as the treatment name 66, hot biopsy or biopsy as an output value. However, the technique of the present disclosure is not limited thereto. In the present modification, a treatment name derivation table 50D1 includes, as input values, the treatment tool identification result 60 other than forceps and the aspect identification result 64 other than burn scar. The treatment name derivation table 50D1 has, as an output value, the treatment name 66 other than hot biopsy or biopsy.

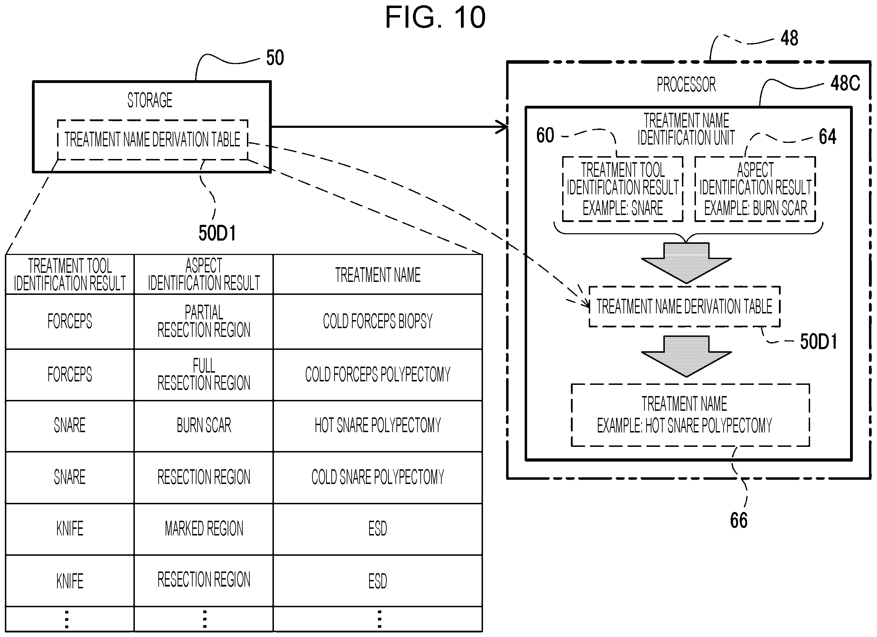

As illustrated as an example in Fig. 10, the treatment name derivation table 50D1 has, as the treatment tool identification result 60, forceps as an input value, and further has, as the aspect identification result 64, a region in which a target portion in the body has been partially resected (i.e., a partial resection region) as an input value. In this case, the treatment name derivation table 50D1 has, as the treatment name 66, cold forceps biopsy as an output value. The treatment name derivation table 50D1 has, as the treatment tool identification result 60, forceps as an input value, and further has, as the aspect identification result 64, a region in which a target portion in the body has been fully resected (i.e., a full resection region) as an input value. In this case, the treatment name derivation table 50D 1 has, as the treatment name 66, cold forceps polypectomy as an output value.

The treatment name derivation table 50D1 has, as the treatment tool identification result 60, snare as an input value, and further has, as the aspect identification result 64, burn scar as an input value. In this case, the treatment name derivation table 50D1 has, as the treatment name 66, hot snare polypectomy as an output value. The treatment name derivation table 50D1 has, as the treatment tool identification result 60, snare as an input value, and further has, as the aspect identification result 64, resection region as an input value. In this case, the treatment name derivation table 50D1 has, as the treatment name 66, cold snare polypectomy as an output value.

The treatment name derivation table 50D1 has, as the treatment tool identification result 60, knife as an input value, and further has, as the aspect identification result 64, marked region as an input value. In this case, the treatment name derivation table 50D1 has, as the treatment name 66, ESD as an output value. The treatment name derivation table 50D1 has, as the treatment tool identification result 60, knife as an input value, and further has, as the aspect identification result 64, resection region (for example, a submucosal layer after a lesion region has been peeled off) as an input value. In this case, the treatment name derivation table 50D1 has, as the treatment name 66, ESD as an output value.

As described above, in the endoscope apparatus 10 according to the present modification, the treatment name 66 is derived from the treatment name derivation table 50D1. In the treatment name derivation table 50D1, the internal aspect 62 as input values include burn scar, resection region, change in resection region, and marked region. Thus, according to this configuration, it is possible to realize output of the treatment name 66 with higher accuracy than in a case where the treatment name 66 is identified based on only the presence or absence of the internal aspect 62.

### Modification 2

In the above-described embodiment, a description has been given of an example in which the treatment name derivation table 50D is a table having the type of the treatment tool 58 (see Fig. 4) included in the endoscopic image 56 (see Fig. 4) and the internal aspect 62 (see Fig. 6) included in the endoscopic image 56 as input values and having the treatment name 66 (see Fig. 7) as an output value, but the technique of the present disclosure is not limited thereto. In the present modification, a treatment name derivation table 50D2 has a treatment tool size 61 and an aspect size 65 as input values in addition to the treatment tool identification result 60 and the aspect identification result 64. The treatment tool size 61 is the size of the treatment tool 58, and the aspect size 65 is the size of the internal aspect 62.

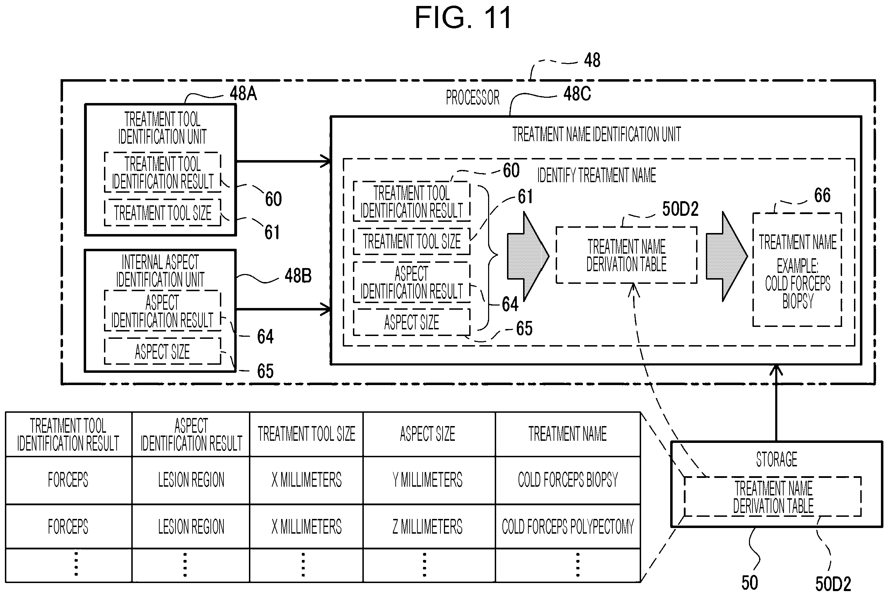

As illustrated as an example in Fig. 11, the treatment name derivation table 50D2 has the treatment tool identification result 60, the aspect identification result 64, the treatment tool size 61, and the aspect size 65 as input values. The treatment tool size 61 is derived by using the treatment tool identifying NN 50B at the time of identifying the type of the treatment tool 58 included in the endoscopic image 56 by the treatment tool identification unit 48A. The aspect size 65 is derived by using the internal aspect identifying NN 50C at the time of identifying the internal aspect 62 included in the endoscopic image 56 by the internal aspect identification unit 48B. The treatment name derivation table 50D2 has, as an output value, the treatment name 66 that is related to the treatment tool identification result 60, the aspect identification result 64, the treatment tool size 61, and the aspect size 65.

The treatment name derivation table 50D2 has, as the treatment tool identification result 60, forceps as an input value, and has, as the aspect identification result 64, lesion region as an input value. In this case, the treatment name derivation table 50D2 has, as input values, the size of forceps, which is the treatment tool 58, and the size of a lesion region, which is the internal aspect 62. In a case where the size of the forceps is smaller than the size of the lesion region (for example, in a case where X < Y holds when the size of the forceps is X millimeters and the size of the lesion region is Y millimeters), the treatment name derivation table 50D2 has, as the treatment name 66, cold forceps biopsy as an output value. In a case where the size of the forceps is larger than the size of the lesion region (for example, in a case where X > Z holds when the size of the forceps is X millimeters and the size of the lesion region is Z millimeters), the treatment name derivation table 50D2 has, as the treatment name 66, cold forceps polypectomy as an output value.

As described above, in the endoscope apparatus 10 according to the present modification, the internal aspect 62 includes a lesion region. The information indicating the treatment name 66 is information that is based on the treatment tool size 61 and the aspect size 65. Thus, according to this configuration, it is possible to realize output of the treatment name 66 with higher accuracy than in a case where the treatment name 66 is output based on only a result of identifying the type of the treatment tool 58.

### Modification 3

In the above-described embodiment, a description has been given of an example in which the treatment name derivation table 50D is a table having the type of the treatment tool 58 (see Fig. 4) included in the endoscopic image 56 (see Fig. 4) and the internal aspect 62 (see Fig. 6) included in the endoscopic image 56 as input values and having the treatment name 66 (see Fig. 7) as an output value, but the technique of the present disclosure is not limited thereto. In the present modification, a treatment name derivation table 50D3 has, as an input value, the ratio of the treatment tool size 61 to the aspect size 65, in addition to the treatment tool identification result 60 and the aspect identification result 64.

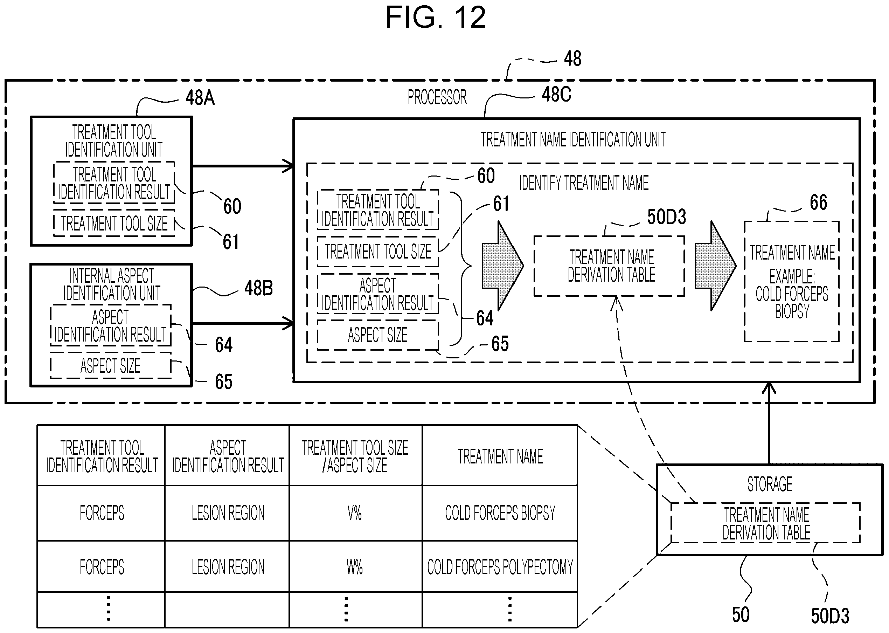

As illustrated as an example in Fig. 12, the treatment name derivation table 50D3 has, as input values, the treatment tool identification result 60, the aspect identification result 64, and the ratio of the treatment tool size 61 to the aspect size 65. The ratio of the treatment tool size 61 to the aspect size 65 is calculated by the treatment name identification unit 48C, based on the treatment tool size 61 and the aspect size 65. The treatment name derivation table 50D2 has, as an output value, the treatment name 66 that is related to the treatment tool identification result 60, the aspect identification result 64, and the ratio of the treatment tool size 61 to the aspect size 65.

The treatment name derivation table 50D3 has, as the treatment tool identification result 60, forceps as an input value, and has, as the aspect identification result 64, lesion region as an input value. In this case, the treatment name derivation table 50D3 has, as an input value, the ratio between the size of forceps, which is the treatment tool 58, and the size of a lesion region, which is the internal aspect 62. In a case where the size of the forceps is smaller than the size of the lesion region (for example, in a case where (treatment tool size/aspect size) × 100 = V(%) and V < 100 holds), the treatment name derivation table 50D3 has, as the treatment name 66, cold forceps biopsy as an output value. In a case where the size of the forceps is larger than the size of the lesion region (for example, in a case where (treatment tool size/aspect size) × 100 = W(%) and W ≥ 100 holds), the treatment name derivation table 50D3 has, as the treatment name 66, cold forceps polypectomy as an output value.

As described above, in the endoscope apparatus 10 according to the present modification, the internal aspect 62 includes a lesion region, and the information indicating the treatment name 66 is information that is based on the ratio of the treatment tool size 61 to the aspect size 65. Thus, according to this configuration, it is possible to realize output of the treatment name 66 with higher accuracy than in a case where the treatment name 66 is output based on only a result of identifying the type of the treatment tool 58.

### Modification 4

In the above-described first embodiment, a description has been given of an example in which, in the case of identifying the internal aspect 62, the internal aspect 62 is identified based on the endoscopic image 56B including the internal aspect 62, but the technique of the present disclosure is not limited thereto. In the present modification, the internal aspect 62 may be identified based on an identification result of the treatment tool 58 together with the endoscopic image 56B.

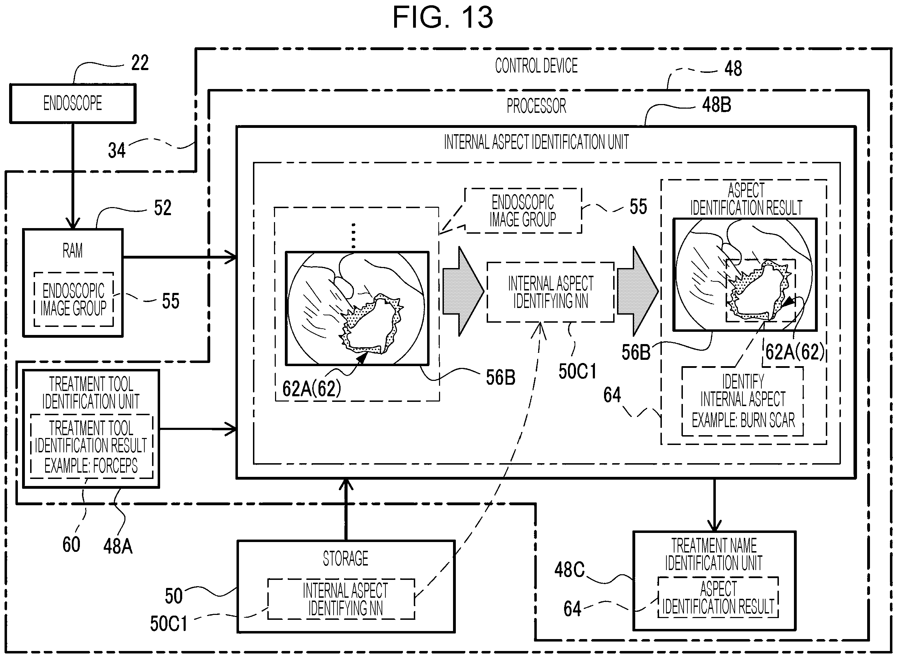

As illustrated as an example in Fig. 13, the internal aspect identification unit 48B acquires the endoscopic image group 55 from the RAM 52. The endoscopic image group 55 includes the endoscopic image 56B in which the internal aspect 62 is seen. The internal aspect identification unit 48B also acquires the treatment tool identification result 60 from the treatment tool identification unit 48A. Fig. 13 illustrates an example in which the forceps 32 are identified as the treatment tool 58.

The internal aspect identification unit 48B identifies, based on the endoscopic image 56B and the treatment tool identification result 60, the type of the internal aspect 62 included in the endoscopic image 56B. For example, the internal aspect identification unit 48B performs an image recognition process using AI to identify the internal aspect 62.

The storage 50 stores an internal aspect identifying NN 50C1. The internal aspect identifying NN 50C1 is an NN for identifying the internal aspect 62 included in the endoscopic image 56 in accordance with the type of the treatment tool 58, and is a trained model generated by optimizing an NN through machine learning. The internal aspect identifying NN 50C1 is, for example, a trained model optimized for identifying the internal aspect 62 related to the forceps 32.

The internal aspect identification unit 48B acquires, based on the treatment tool identification result 60, the internal aspect identifying NN 50C1 from the storage 50. The internal aspect identification unit 48B identifies the internal aspect 62 by using the internal aspect identifying NN 50C. The treatment name identification unit 48C acquires the aspect identification result 64 from the internal aspect identification unit 48B.

As described above, in the endoscope apparatus 10 according to the present modification, the internal aspect 62 is identified based on the endoscopic image 56B including the internal aspect 62 and the treatment tool identification result 60. Thus, according to this configuration, it is possible to realize output of the treatment name 66 with higher accuracy than in a case where the internal aspect 62 is identified based on only the endoscopic image 56B.

### Second Embodiment

In the above-described first embodiment, a description has been given of an example in which the treatment name 66 is identified in accordance with the treatment tool identification result 60 and the aspect identification result 64, but the technique of the present disclosure is not limited thereto. In a second embodiment, the treatment name 66 is identified in accordance with the identification results of two types of treatment tools 58.

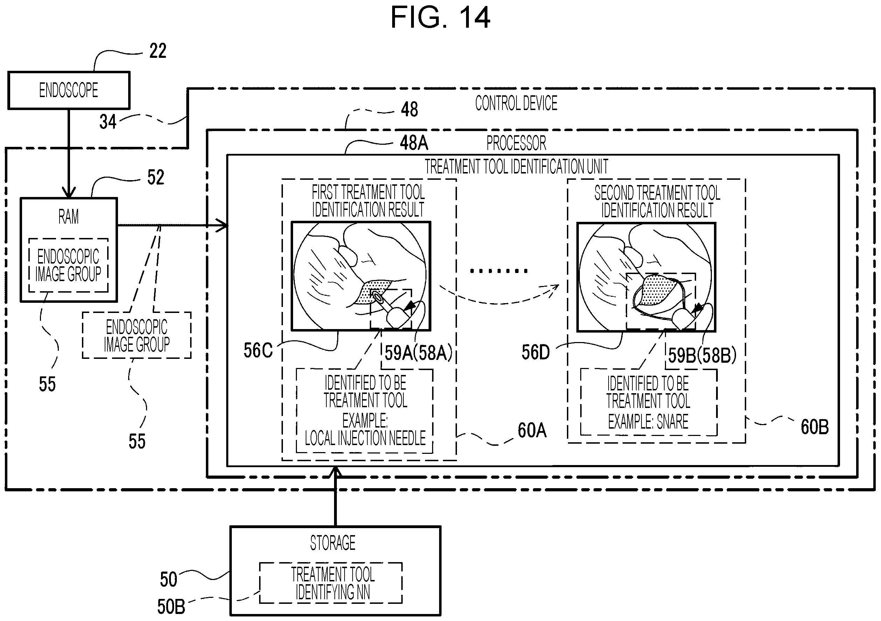

As illustrated as an example in Fig. 14, the treatment tool identification unit 48A acquires the endoscopic image group 55 from the RAM 52. The endoscopic image group 55 includes an endoscopic image 56C including a first treatment tool 58A and an endoscopic image 56D including a second treatment tool 58B. The treatment tool identification unit 48A identifies the type of the first treatment tool 58A included in the endoscopic image 56C by using the treatment tool identifying NN 50B. Fig. 14 illustrates an example in which the type of the first treatment tool 58A is identified to be a local injection needle 59A as a first treatment tool identification result 60A. The endoscopic image 56C is an example of a "first endoscopic image" according to the technique of the present disclosure, the first treatment tool 58A is an example of a "first treatment tool" according to the technique of the present disclosure, and the first treatment tool identification result 60A is an example of a "first identification result" according to the technique of the present disclosure.

In a case where a predetermined condition is satisfied after the type of the first treatment tool 58A is identified as the first treatment tool identification result 60A, the treatment tool identification unit 48A performs the subsequent identification of the type of the second treatment tool 58B. For example, the predetermined condition is that the second treatment tool 58B is seen in the endoscopic image 56D. The treatment tool identification unit 48A identifies the type of the second treatment tool 58B included in the endoscopic image 56 by using the treatment tool identifying NN 50B. Fig. 14 illustrates an example in which the type of the second treatment tool 58B is identified to be a snare 59B as a second treatment tool identification result 60B. The endoscopic image 56D is an example of a "second endoscopic image" according to the technique of the present disclosure, the second treatment tool identification result 60B is an example of a "second identification result" according to the technique of the present disclosure, and the second treatment tool 58B is an example of a "second treatment tool" according to the technique of the present disclosure.

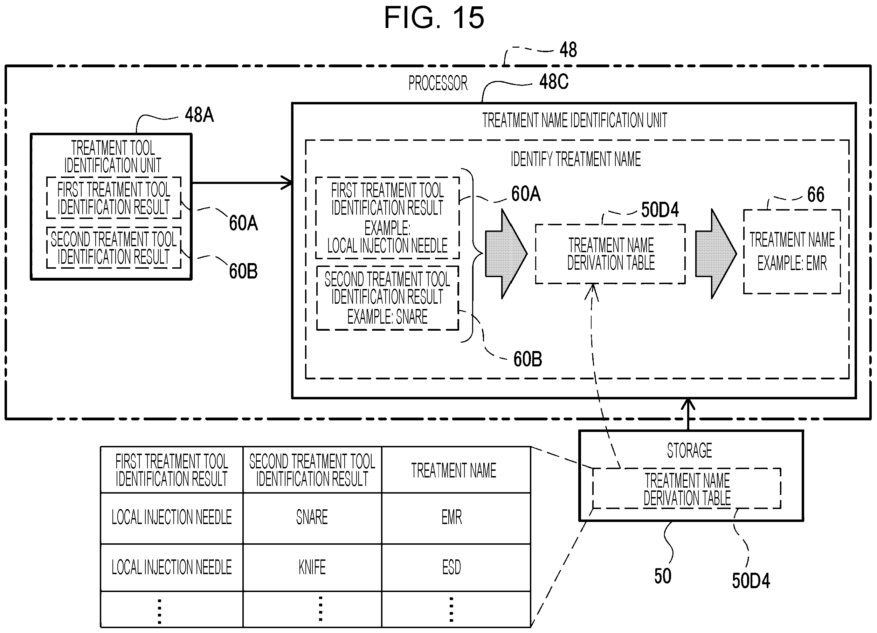

As illustrated as an example in Fig. 15, the treatment name identification unit 48C acquires the first treatment tool identification result 60A and the second treatment tool identification result 60B from the treatment tool identification unit 48A. The treatment name identification unit 48C derives, from a treatment name derivation table 50D4, the treatment name 66 that is related to the first treatment tool identification result 60A and the second treatment tool identification result 60B. In the example illustrated in Fig. 15, "local injection needle" is identified as the first treatment tool identification result 60A, and "snare" is identified as the second treatment tool identification result 60B. The treatment name identification unit 48C derives, from the treatment name derivation table 50D4, "EMR" as the treatment name 66 that is related to "local injection needle", which is the first treatment tool identification result 60A, and "snare", which is the second treatment tool identification result 60B.

The treatment name derivation table 50D4 has, as the first treatment tool identification result 60, local injection needle as an input value, and further has, as the second treatment tool identification result 60B, knife as an input value. In this case, the treatment name derivation table 50D4 has, as the treatment name 66, ESD as an output value.

As described above, in the endoscope apparatus 10 according to the second embodiment, information indicating the treatment name 66 is output based on the first treatment tool identification result 60A, which is a result of identifying the type of the first treatment tool 58A, and the second treatment tool identification result 60B, which is a result of identifying the type of the second treatment tool 58B. Thus, according to this configuration, it is possible to realize output of the treatment name 66 with higher accuracy than in a case where the treatment name 66 is output based on only a result of identifying the type of the first treatment tool 58A.

### Third Embodiment

In the above-described first embodiment, a description has been given of an example in which the treatment name 66 is identified in accordance with the treatment tool identification result 60 and the aspect identification result 64, but the technique of the present disclosure is not limited thereto. In a third embodiment, the treatment name 66 is identified in accordance with the identification results of the two types of treatment tools 58 and the identification result of the internal aspect 62.

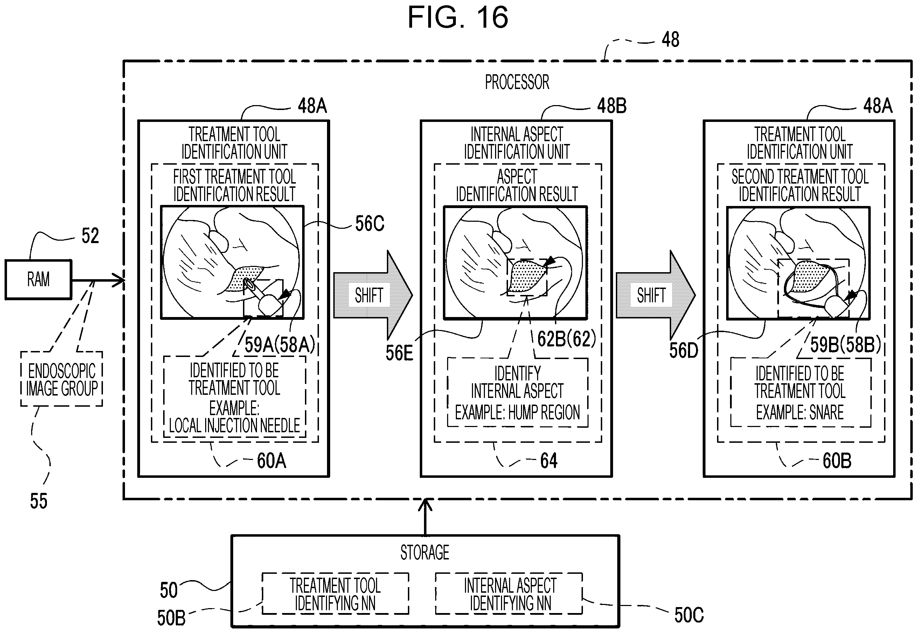

As illustrated as an example in Fig. 16, the treatment tool identification unit 48A acquires the endoscopic image group 55 from the RAM 52. The endoscopic image group 55 includes the endoscopic image 56C including the first treatment tool 58A and the endoscopic image 56D including the second treatment tool 58B. The treatment tool identification unit 48A identifies the type of the first treatment tool 58A included in the endoscopic image 56 by using the treatment tool identifying NN 50B. Fig. 16 illustrates an example in which the type of the first treatment tool 58A is identified to be the local injection needle 59A as the first treatment tool identification result 60A. The endoscopic image 56C is an example of a "first endoscopic image" according to the technique of the present disclosure, the first treatment tool 58A is an example of a "first treatment tool" according to the technique of the present disclosure, and the first treatment tool identification result 60A is an example of a "first identification result" according to the technique of the present disclosure.

In a case where a predetermined condition is satisfied after the type of the first treatment tool 58A is identified as the first treatment tool identification result 60A, a shift from the first treatment tool identification process by the treatment tool identification unit 48A to the internal aspect identification process by the internal aspect identification unit 48B occurs. For example, the predetermined condition is that the type of the first treatment tool 58A is not identified in a plurality of frames (for example, two to three frames) after the type of the first treatment tool 58A is identified.

The internal aspect identification unit 48B acquires the endoscopic image group 55 from the RAM 52. The endoscopic image group 55 includes an endoscopic image 56E including the internal aspect 62. Here, the internal aspect 62 is generated after the treatment with the first treatment tool 58A. Thus, the endoscopic image 56E is an image acquired after the endoscopic image 56C. The internal aspect identification unit 48B identifies the internal aspect 62 included in the endoscopic image 56E by using the internal aspect identifying NN 50C. Fig. 16 illustrates an example in which the internal aspect 62 is identified to be a hump region 62B (i.e., a region in which a part of the inside of the body is humped as a result of a liquid (for example, normal saline solution) being injected through the local injection needle 59A) as the aspect identification result 64. The endoscopic image 56E is an example of a "second endoscopic image" according to the technique of the present disclosure, the internal aspect 62 is an example of a "first internal aspect" according to the technique of the present disclosure, and the aspect identification result 64 is an example of a "second identification result" according to the technique of the present disclosure.

In a case where a predetermined condition is satisfied after the internal aspect 62 is identified as the aspect identification result 64, a shift from the internal aspect identification process by the internal aspect identification unit 48B to the second treatment tool identification process by the treatment tool identification unit 48A occurs. For example, the predetermined condition is that the second treatment tool 58B is seen in the endoscopic image 56D.

The treatment tool identification unit 48A identifies the type of the second treatment tool 58B included in the endoscopic image 56D by using the treatment tool identifying NN 50B. Here, the internal aspect 62 is generated after the treatment with the first treatment tool 58A, and the second treatment tool 58B is used for the internal aspect 62. Thus, the endoscopic image 56D is an image acquired after the endoscopic image 56E.

Fig. 16 illustrates an example in which the type of the second treatment tool 58B is identified to be the snare 59B as the second treatment tool identification result 60B. The endoscopic image 56E is an example of a "third endoscopic image" according to the technique of the present disclosure, the second treatment tool 58B is an example of a "third treatment tool" according to the technique of the present disclosure, and the second treatment tool identification result 60B is an example of a "third identification result" according to the technique of the present disclosure.

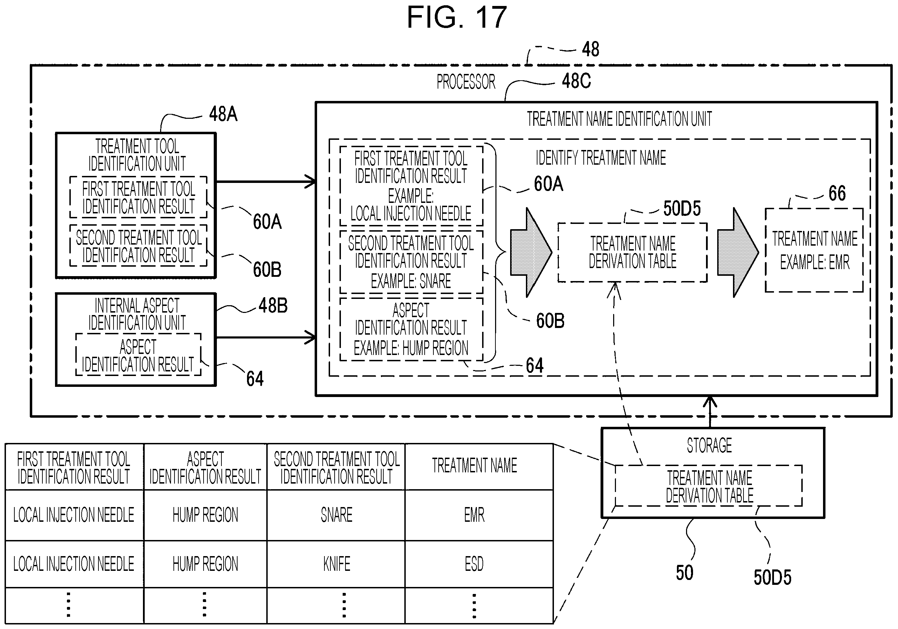

As illustrated as an example in Fig. 17, the treatment name identification unit 48C acquires the first treatment tool identification result 60A and the second treatment tool identification result 60B from the treatment tool identification unit 48A. The treatment name identification unit 48C also acquires the aspect identification result 64 from the internal aspect identification unit 48B. The treatment name identification unit 48C derives, from a treatment name derivation table 50D5, the treatment name 66 that is related to the first treatment tool identification result 60A and the second treatment tool identification result 60B. In the example illustrated in Fig. 17, "local injection needle" is identified as the first treatment tool identification result 60A, and "snare" is identified as the second treatment tool identification result 60B. In addition, "hump region" is identified as the aspect identification result 64. The treatment name identification unit 48C derives, from the treatment name derivation table 50D4, "EMR" as the treatment name 66 that is related to the first treatment tool identification result 60A, the second treatment tool identification result 60B, and the aspect identification result 64.

The treatment name derivation table 50D5 has, as the first treatment tool identification result 60, local injection needle as an input value. The treatment name derivation table 50D5 has, as the aspect identification result 64, hump region as an input value. Furthermore, the treatment name derivation table 50D5 has, as the second treatment tool identification result 60B, knife as an input value. In this case, the treatment name derivation table 50D4 has, as the treatment name 66, ESD as an output value.

As described above, in the endoscope apparatus 10 according to the third embodiment, the information indicating the treatment name 66 is information that is related to the first treatment tool identification result 60A, which is a result of identifying the type of the first treatment tool 58A, the aspect identification result 64, which is a result of identifying the internal aspect 62, and the second treatment tool identification result 60B, which is a result of identifying the type of the second treatment tool 58B. Thus, according to this configuration, it is possible to realize output of the treatment name 66 with higher accuracy than in a case where the treatment name 66 is output based on only a result of identifying the type of the first treatment tool 58A.

In the endoscope apparatus 10 according to the third embodiment, the endoscopic image 56D including the second treatment tool 58B is acquired by the endoscope 22 after the endoscopic image 56E including the internal aspect 62 is acquired. Thus, according to this configuration, it is possible to realize output of the treatment name 66 with higher accuracy than in a case where the order in which the endoscopic image 56D and the endoscopic image 56E are acquired is not taken into consideration in the case of identifying the treatment name 66.

### Modification 5

In the above-described third embodiment, a description has been given of an example in which identification is performed in the order of the first treatment tool 58A (for example, the local injection needle 59A), the internal aspect 62 (for example, the hump region 62B), and the second treatment tool 58B (for example, the snare 59B), but the technique of the present disclosure is not limited thereto. In the present modification, identification is performed in the order of the first treatment tool 58A, the second treatment tool 58B, and the internal aspect 62.

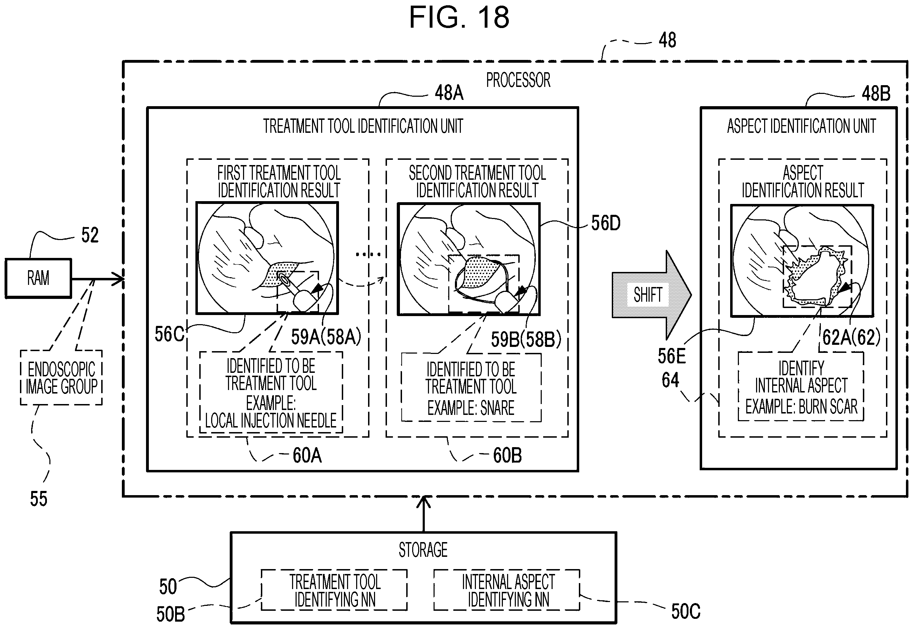

As illustrated as an example in Fig. 18, the treatment tool identification unit 48A acquires the endoscopic image group 55 from the RAM 52. The endoscopic image group 55 includes the endoscopic image 56C including the first treatment tool 58A and the endoscopic image 56D including the second treatment tool 58B. Here, the second treatment tool 58B is used after the treatment with the first treatment tool 58A. Thus, the endoscopic image 56D is an image acquired after the endoscopic image 56C.

The treatment tool identification unit 48A identifies the type of the first treatment tool 58A included in the endoscopic image 56C by using the treatment tool identifying NN 50B. The endoscopic image 56C is an example of a "first endoscopic image" according to the technique of the present disclosure, the first treatment tool 58A is an example of a "first treatment tool" according to the technique of the present disclosure, and the first treatment tool identification result 60A is an example of a "first identification result" according to the technique of the present disclosure.

In a case where a predetermined condition (for example, the second treatment tool 58B is seen in the endoscopic image 56D) is satisfied after the type of the first treatment tool 58A is identified as the first treatment tool identification result 60A, the treatment tool identification unit 48A identifies the type of the second treatment tool 58B included in the endoscopic image 56D by using the treatment tool identifying NN 50B. The endoscopic image 56D is an example of a "second endoscopic image" according to the technique of the present disclosure, the second treatment tool 58B is an example of a "second treatment tool" according to the technique of the present disclosure, and the second treatment tool identification result 60B is an example of a "second identification result" according to the technique of the present disclosure.

In a case where a predetermined condition is satisfied after the type of the second treatment tool 58B is identified, a shift from the treatment tool identification process by the treatment tool identification unit 48A to the internal aspect identification process by the internal aspect identification unit 48B occurs. For example, the predetermined condition is that the type of the second treatment tool 58B is not identified in a plurality of frames (for example, two to three frames) after the type of the second treatment tool 58B is identified.

The internal aspect identification unit 48B acquires the endoscopic image group 55 from the RAM 52. The endoscopic image group 55 includes the endoscopic image 56E including the internal aspect 62. Here, the internal aspect 62 is generated after the treatment with the second treatment tool 58B. Thus, the endoscopic image 56E is an image acquired after the endoscopic image 56D.

The internal aspect identification unit 48B identifies the internal aspect 62 included in the endoscopic image 56 by using the internal aspect identifying NN 50C. The endoscopic image 56E is an example of a "third endoscopic image" according to the technique of the present disclosure, the internal aspect 62 is an example of a "second internal aspect" according to the technique of the present disclosure, and the aspect identification result 64 is an example of a "third identification result" according to the technique of the present disclosure.

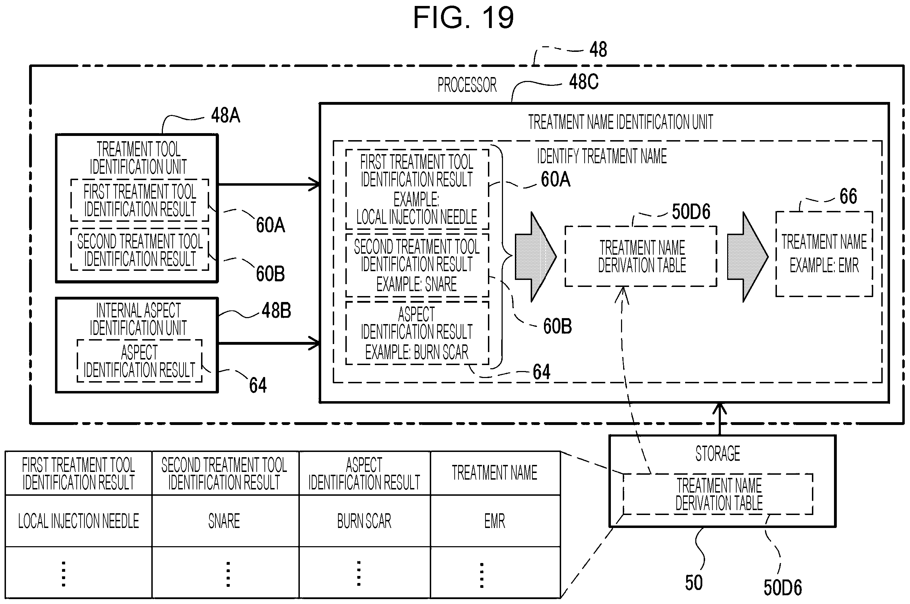

As illustrated as an example in Fig. 19, the treatment name identification unit 48C derives, from a treatment name derivation table 50D6, the treatment name 66 that is related to the first treatment tool identification result 60A and the second treatment tool identification result 60B. In the example illustrated in Fig. 19, "local injection needle" is identified as the first treatment tool identification result 60A, and "snare" is identified as the second treatment tool identification result 60B. In addition, "burn scar" is identified as the aspect identification result 64. The treatment name identification unit 48C derives, from the treatment name derivation table 50D6, "EMR" as the treatment name 66 that is related to the first treatment tool identification result 60A, the second treatment tool identification result 60B, and the aspect identification result 64.

As described above, in the endoscope apparatus 10 according to the present modification, the information indicating the treatment name 66 is information that is related to the first treatment tool identification result 60A, which is a result of identifying the type of the first treatment tool 58A, the second treatment tool identification result 60B, which is a result of identifying the type of the second treatment tool 58B, and the aspect identification result 64, which is a result of identifying the internal aspect 62. Thus, according to this configuration, it is possible to realize output of the treatment name 66 with higher accuracy than in a case where the treatment name 66 is output based on only a result of identifying the type of the first treatment tool 58A.

In the endoscope apparatus 10 according to the present modification, the endoscopic image 56E including the internal aspect 62 is acquired by the endoscope 22 after the endoscopic image 56D including the second treatment tool 58B is acquired. Thus, according to this configuration, it is possible to realize output of the treatment name 66 with higher accuracy than in a case where the order in which the endoscopic image 56D and the endoscopic image 56E are acquired is not taken into consideration in the case of identifying the treatment name 66.

### Fourth Embodiment

In the above-described first embodiment, a description has been given of an example in which identification is performed in the order of the treatment tool 58 and the internal aspect 62, but the technique of the present disclosure is not limited thereto. In a fourth embodiment, identification is performed in the order of the internal aspect 62 and the treatment tool 58.

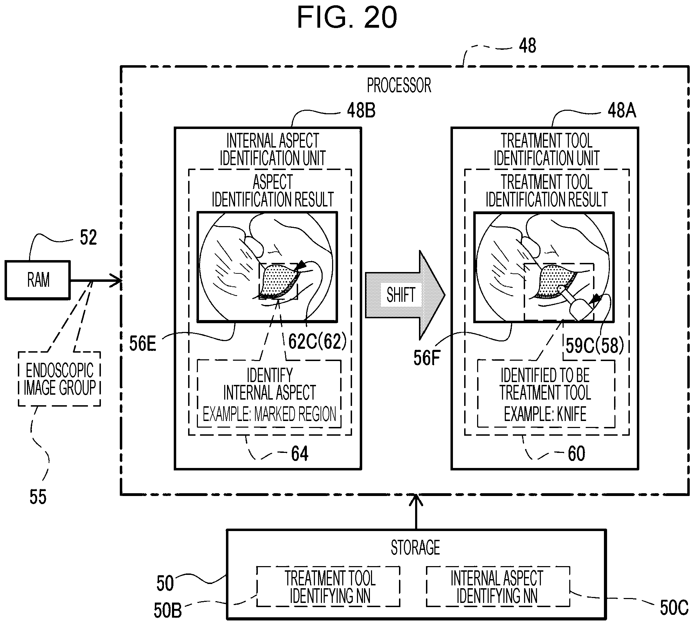

As illustrated as an example in Fig. 20, the internal aspect identification unit 48B acquires the endoscopic image group 55 from the RAM 52. The endoscopic image group 55 includes the endoscopic image 56E including the internal aspect 62. The internal aspect identification unit 48B identifies the internal aspect 62 included in the endoscopic image 56 by using the internal aspect identifying NN 50C. Fig. 20 illustrates an example in which the internal aspect 62 is identified to be a marked region 62C as a treatment target region (for example, a region in which a target portion in the body is marked with a pigment) as the aspect identification result 64. The marked region 62C is an example of a "treatment target region" according to the technique of the present disclosure.

In a case where a predetermined condition is satisfied after the internal aspect 62 is identified as the aspect identification result 64, a shift from the internal aspect identification process by the internal aspect identification unit 48B to the treatment tool identification process by the treatment tool identification unit 48A occurs. For example, the predetermined condition is that the treatment tool 58 is seen in the endoscopic image 56D.

The internal aspect identification unit 48B acquires the endoscopic image group 55 from the RAM 52. The endoscopic image group 55 includes an endoscopic image 56F including the treatment tool 58. Here, after a treatment target region is determined, a treatment with the treatment tool 58 (for example, resection of the treatment target region) is performed. Thus, the endoscopic image 56F is an image acquired after the endoscopic image 56E. The treatment tool identification unit 48A identifies the type of the treatment tool 58 included in the endoscopic image 56F by using the treatment tool identifying NN 50B. Fig. 20 illustrates an example in which the treatment tool 58 is identified to be a knife 59C as the treatment tool identification result 60.

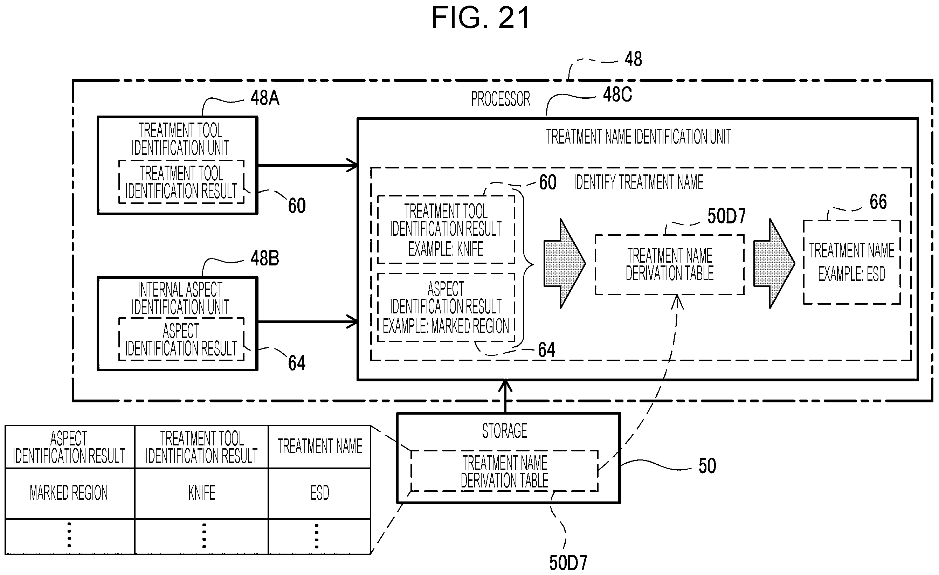

As illustrated as an example in Fig. 21, the treatment name identification unit 48C derives, from a treatment name derivation table 50D7, the treatment name 66 that is related to the aspect identification result 64 and the treatment tool identification result 60. In the example illustrated in Fig. 21, "knife" is identified as the treatment tool identification result 60, and "marked region" is identified as the aspect identification result 64. For example, a marked region is a region in which a resection target portion is marked in the body by using a knife. The treatment name identification unit 48C derives, from the treatment name derivation table 50D7, "ESD" as the treatment name 66 that is related to the treatment tool identification result 60 and the aspect identification result 64.

As described above, in the endoscope apparatus 10 according to the fourth embodiment, information indicating the treatment name 66 is output based on the aspect identification result 64, which is a result of identifying the internal aspect 62, and the treatment tool identification result 60, which is a result of identifying the type of the treatment tool 58. Thus, according to this configuration, it is possible to realize output of the treatment name 66 with higher accuracy than in a case where the treatment name 66 is output based on only a result of identifying the type of the treatment tool 58.

In the endoscope apparatus 10 according to the fourth embodiment, the endoscopic image 56F including the treatment tool 58 is acquired by the endoscope 22 after the endoscopic image 56E including the internal aspect 62 is acquired. Thus, according to this configuration, it is possible to realize output of the treatment name 66 with higher accuracy than in a case where the order in which the endoscopic image 56E and the endoscopic image 56F are acquired is not taken into consideration in the case of identifying the treatment name 66.

In the endoscope apparatus 10 according to the fourth embodiment, the marked region 62C, which is a treatment target region, is identified as the internal aspect 62. Thus, according to this configuration, it is possible to realize output of the treatment name 66 with higher accuracy than in a case where the treatment name 66 is identified based on only the presence or absence of the internal aspect 62.

In each of the above-described embodiments, a description has been given of an example in which the type of the treatment tool 58 is identified or the internal aspect 62 is identified by an image recognition process using the AI method, but the technique of the present disclosure is not limited thereto. For example, an image recognition process using a pattern matching method may be performed.

In each of the above-described embodiments and each of the above-described modifications, a description has been given of an example in which the treatment name derivation tables 50D and 50D1 to 50D7 are different tables, but the technique of the present disclosure is not limited thereto. The treatment name derivation tables 50D and 50D1 to 50D7 may be combined into one table.

In each of the above-described embodiments, a description has been given of an example in which one treatment is performed and the treatment name 66 is identified in one examination or remedy, but the technique of the present disclosure is not limited thereto. For example, in one examination or remedy, a plurality of treatments may be performed on a plurality of portions in the body, and the treatment name 66 may be identified for each of the plurality of treatments.

In each of the above-described embodiments, a description has been given of an example in which an image recognition process is performed on the endoscopic images 56 acquired during an examination or remedy to identify the treatment name 66, but the technique of the present disclosure is not limited thereto. For example, an image recognition process may be performed on the endoscopic images 56 acquired in a past examination or remedy to identify the treatment name 66.

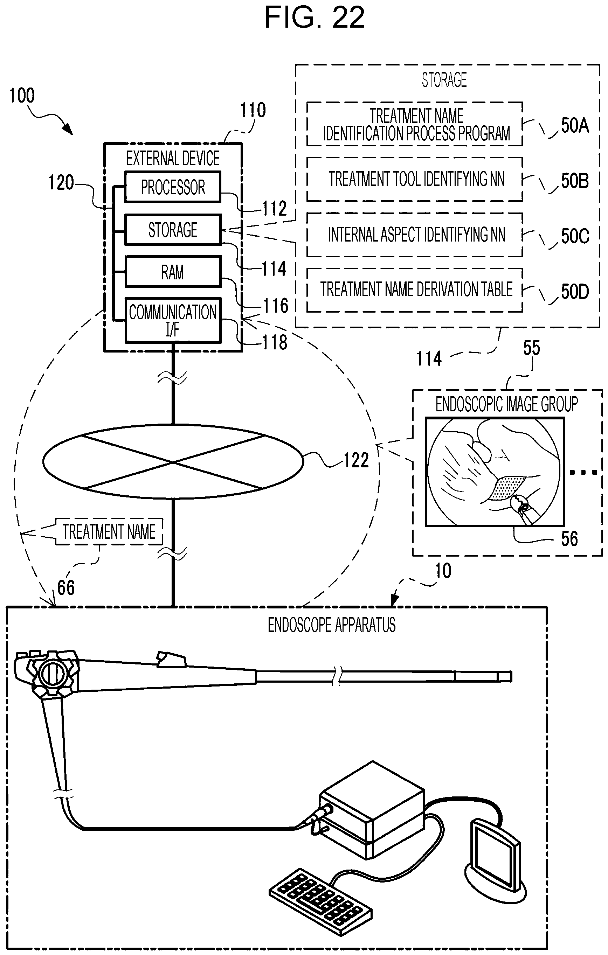

In each of the above-described embodiments, a description has been given of an example in which a treatment name identification process is performed by the processor 48 of the control device 34 included in the endoscope apparatus 10, but the technique of the present disclosure is not limited thereto. A device that performs a treatment name identification process may be provided outside the endoscope apparatus 10. In this case, an endoscope system 100 may be used as illustrated as an example in Fig. 22. The endoscope system 100 includes the endoscope apparatus 10 and an external device 110. The external device 110 is, for example, a server. The server is implemented by, for example, cloud computing. Here, cloud computing is illustrated, but this is merely an example. For example, the server may be implemented by a mainframe, or may be implemented by network computing such as fog computing, edge computing, or grid computing. Here, a server is given as an example of the external device 110, but this is merely an example. At least one personal computer or the like may be used as the external device 110 instead of the server.

The external device 110 includes a processor 112, a storage 114, a RAM 116, and a communication I/F 118. The processor 112, the storage 114, the RAM 116, and the communication I/F 118 are connected to each other by a bus 120. The communication I/F 118 is connected to the endoscope apparatus 10 via a network 122. The network 122 is, for example, the Internet. The network 122 is not limited to the Internet, and may be a WAN and/or a LAN such as an intranet.

The storage 114 stores the treatment name identification process program 50A, the treatment tool identifying NN 50B, the internal aspect identifying NN 50C, and the treatment name derivation table 50D. The processor 112 executes the treatment name identification process program 50Ain the RAM 116. The processor 112 performs the above-described treatment name identification process in accordance with the treatment name identification process program 50A executed on the RAM 116. In the case of performing the treatment name identification process, the processor 112 processes the endoscopic images 56 by using the treatment tool identifying NN 50B and the internal aspect identifying NN 50C as described in each of the above-described examples. The endoscopic image group 55 including a plurality of endoscopic images 56 is transmitted, for example, from the endoscope apparatus 10 to the external device 110 via the network 122. The communication I/F 118 of the external device 110 receives the endoscopic image group 55. The processor 112 performs a treatment name identification process, based on the endoscopic images 56 received by the communication I/F 118. The processor 112 performs the treatment name identification process to generate information indicating the treatment name 66, and transmits the information indicating the treatment name 66 to the endoscope apparatus 10. The endoscope apparatus 10 receives the information indicating the treatment name 66 transmitted from the external device 110.

In the example illustrated in Fig. 22, the external device 110 is an example of a "medical assistance device" according to the technique of the present disclosure, and the processor 112 is an example of a "processor" according to the technique of the present disclosure.

The treatment name identification process may be performed in a distributed manner by a plurality of apparatuses including the endoscope apparatus 10 and the external device 110.

In the above-described embodiments, a description has been given of an example in which the treatment name identification process program 50A is stored in the storage 50, but the technique of the present disclosure is not limited thereto. For example, the treatment name identification process program 50Amay be stored in a portable storage medium such as an SSD or a USB memory. The storage medium is a non-transitory computer-readable storage medium. The treatment name identification process program 50A stored in the storage medium is installed into the computer 44 of the control device 34. The processor 48 executes a treatment name identification process in accordance with the treatment name identification process program 50A.

Although the computer 44 is illustrated in the above-described embodiments, the technique of the present disclosure is not limited thereto. A device including an ASIC, an FPGA, and/or a PLD may be applied instead of the computer 44. Alternatively, a combination of a hardware configuration and a software configuration may be used instead of the computer 44.

In addition, as hardware resources that execute the treatment name identification process described in the above-described embodiments, the following various types of processors can be used. The processors include, for example, a processor which is a general-purpose processor that executes software, that is, a program, to function as hardware resources for executing the treatment name identification process. The processors include, for example, a dedicated electronic circuit, which is a processor having a circuit configuration designed specifically for performing specific processing, such as an FPGA, a PLD, or an ASIC. A memory is built in or connected to each of the processors, and each of the processors executes the treatment name identification process by using the memory.

The hardware resources that execute the treatment name identification process may be constituted by one of these various types of processors or may be constituted by a combination of two or more processors of the same type or different types (for example, a combination of a plurality of FPGAs, or a combination of a processor and an FPGA). The hardware resources that execute the treatment name identification process may be one processor.

In a first example of the configuration using one processor, a combination of one or more processors and software constitute one processor, and the processor functions as hardware resources for executing the treatment name identification process. In a second example, as represented by SoC or the like, a processor in which a single IC chip implements the function of an entire system including a plurality of hardware resources for executing the treatment name identification process is used. In this way, the treatment name identification process is implemented by using one or more of the above-described various types of processors as hardware resources.

Furthermore, as the hardware structure of the various types of processors, more specifically, an electronic circuit formed by combining circuit elements such as semiconductor elements may be used. The above-described estimation process is merely an example. Thus, it goes without saying that an unnecessary step may be deleted, a new step may be added, or the processing order may be changed without departing from the gist.

The description given above and the illustration in the drawings are detailed description of the part according to the technique of the present disclosure and are merely examples of the technique of the present disclosure. For example, the description about the configurations, functions, operations, and effects given above is the description about an example of the configurations, functions, operations, and effects of the part according to the technique of the present disclosure. Thus, it goes without saying that an unnecessary part may be deleted from, a new element may be added to, or replacement may be performed on the description given above and the illustration in the drawings without departing from the gist of the technique of the present disclosure. To avoid complexity and facilitate understanding of the part according to the technique of the present disclosure, description of common technical knowledge or the like that is not particularly necessary to implement the technique of the present disclosure is omitted in the description given above and the illustration in the drawings.

In this specification, "A and/or B" is synonymous with "at least one of A or B". That is, "A and/or B" means only A, only B, or a combination of A and B. In this specification, a concept similar to "A and/or B" is applied to three or more things connected by "and/or".

All documents, patent applications, and technical standards described in this specification are incorporated in this specification by reference to such a degree that each document, patent application, and technical standard are specifically and individually described as being incorporated by reference.

The disclosure of JP2022-014192 filed on February 1, 2022 is incorporated herein by reference in its entirety.

## Claims

1. A medical assistance device comprising:
a processor configured to:
acquire a plurality of endoscopic images acquired by capturing images in time series by an endoscope;
identify, based on a first endoscopic image of the plurality of endoscopic images, a type of a first treatment tool included in the first endoscopic image;
identify a type of a second treatment tool included in a second endoscopic image of the plurality of endoscopic images or a first internal aspect included in the second endoscopic image; and
output information indicating a treatment name that is related to a first identification result and a second identification result, the first identification result being a result of identifying the type of the first treatment tool, the second identification result being a result of identifying the type of the second treatment tool or the first internal aspect.

2. The medical assistance device according to claim 1, wherein the second endoscopic image is an image acquired after the first endoscopic image by the endoscope.

3. The medical assistance device according to claim 1 or 2, wherein the processor is configured to identify, based on the first identification result and the second endoscopic image, the first internal aspect.

4. The medical assistance device according to any one of claims 1 to 3, wherein the processor is configured to, in a case where the type of the first treatment tool is not identified in a plurality of frames after the type of the first treatment tool is identified, identify the type of the second treatment tool or the first internal aspect within a time period determined in accordance with the type of the first treatment tool.

5. The medical assistance device according to any one of claims 1 to 4, wherein
the processor is configured to output an output image associated with the treatment name, and
the output image is an image acquired between the first endoscopic image and the second endoscopic image of the plurality of images.

6. The medical assistance device according to any one of claims 1 to 5, wherein
the processor is configured to further identify a type of a third treatment tool included in a third endoscopic image or a second internal aspect included in the third endoscopic image, and
the information indicating the treatment name is information that is related to the first identification result, the second identification result, and a third identification result, the third identification result being a result of identifying the type of the third treatment tool or the second internal aspect.

7. The medical assistance device according to claim 6, wherein the second identification result is a result of identifying the type of the second treatment tool.

8. The medical assistance device according to claim 6, wherein the second identification result is a result of identifying the first internal aspect.

9. The medical assistance device according to any one of claims 6 to 8, wherein the third endoscopic image is an image acquired after the second endoscopic image by the endoscope.

10. The medical assistance device according to claim 1, wherein the first endoscopic image is an image acquired after the second endoscopic image by the endoscope.

11. The medical assistance device according to claim 10, wherein the first internal aspect is a treatment target region.

12. The medical assistance device according to any one of claims 1 to 11, wherein the first internal aspect includes a lesion region, a burn scar, a resection region, a change in the resection region, a hump region, and/or a marked region.

13. The medical assistance device according to any one of claims 1 to 12, wherein
the first internal aspect includes a lesion region, and
the information indicating the treatment name is information that is based on a size of the first treatment tool and a size of the lesion region.

14. An endoscope apparatus comprising:
the medical assistance device according to any one of claims 1 to 13; and
the endoscope.

15. A medical assistance method comprising:
acquiring a plurality of endoscopic images acquired by capturing images in time series by an endoscope;
identifying, based on a first endoscopic image of the plurality of endoscopic images, a type of a first treatment tool included in the first endoscopic image;
identifying a type of a second treatment tool included in a second endoscopic image of the plurality of endoscopic images or a first internal aspect included in the second endoscopic image; and
outputting information indicating a treatment name that is related to a first identification result and a second identification result, the first identification result being a result of identifying the type of the first treatment tool, the second identification result being a result of identifying the type of the second treatment tool or the first internal aspect.

16. A program for causing a computer to execute a process comprising:
acquiring a plurality of endoscopic images acquired by capturing images in time series by an endoscope;
identifying, based on a first endoscopic image of the plurality of endoscopic images, a type of a first treatment tool included in the first endoscopic image;
identifying a type of a second treatment tool included in a second endoscopic image of the plurality of endoscopic images or a first internal aspect included in the second endoscopic image; and
outputting information indicating a treatment name that is related to a first identification result and a second identification result, the first identification result being a result of identifying the type of the first treatment tool, the second identification result being a result of identifying the type of the second treatment tool or the first internal aspect.
